(19) Europäisches Patentamt
European Patent Office
Office européen des brevets

(11) **EP 3 304 494 B1**

(12) # EUROPEAN PATENT SPECIFICATION

(45) Date of publication and mention of the grant of the patent:
**17.06.2020  Bulletin 2020/25**

(21) Application number: **16804484.0**

(22) Date of filing: **03.06.2016**

(51) Int Cl.:
*G06T 7/40* (2017.01)          *G01N 21/78* (2006.01)
*A61B 5/103* (2006.01)

(86) International application number:
**PCT/US2016/035613**

(87) International publication number:
**WO 2016/196862 (08.12.2016 Gazette 2016/49)**

(54) **DEVICES AND METHODS FOR CALIBRATING A COLORIMETRIC SENSOR**

VORRICHTUNG UND VERFAHREN ZUR KALIBRIERUNG EINES KOLORIMETRISCHEN SENSORS

DISPOSITIFS ET PROCÉDÉS D'ÉTALONNAGE D'UN CAPTEUR COLORIMÉTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2015  US 201562171192 P**
                   **14.04.2016  US 201662322623 P**

(43) Date of publication of application:
**11.04.2018  Bulletin 2018/15**

(60) Divisional application:
**20173264.1**

(73) Proprietor: **Palo Alto Health Sciences, Inc.**
**Kirkland, WA 98034 (US)**

(72) Inventors:
  • **GRAHAM, Paul, K.**
    **Danville, CA 94506-1175 (US)**
  • **MOSKOS, Alexander, J.**
    **Danville, CA 94506-1175 (US)**
  • **PLEIS, Jackson, M.**
    **Danville, CA 94506-1175 (US)**

  • **THOMAS, Simon, W.H.**
    **Danville, CA 94506-1175 (US)**

(74) Representative: **J A Kemp LLP**
    **14 South Square**
    **Gray's Inn**
    **London WC1R 5JJ (GB)**

(56) References cited:
    **WO-A1-94/22686          WO-A1-2015/009792**
    **WO-A1-2015/009792     US-A- 4 921 642**
    **US-A- 5 468 451          US-A1- 2006 008 919**
    **US-A1- 2011 146 382     US-A1- 2011 298 844**
    **US-A1- 2013 261 487     US-B1- 6 374 084**

  • **MILLS A ET AL: "Breath-by-breath measurement of carbon dioxide using a plastic film optical sensor", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 39, no. 1-3, 1 March 1997 (1997-03-01), pages 419-425, XP004087783, ISSN: 0925-4005, DOI: 10.1016/S0925-4005(96)02116-8**

**EP 3 304 494 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD

Embodiments described relate generally to quantitative colorimetric capnometry methods and systems.

### BACKGROUND

Methods and devices for measuring quantitative airway carbon dioxide ($CO_2$) gas exchange concentrations and respiratory rate of a subject's breath (capnometry) are well known in the clinical markets. In fact, the use of capnometry during intubated surgical and otherwise critical ventilated patient situations is mandated by standards organizations because it is critical in maintaining safety. By far the most common technology used in commercial instruments is IR spectroscopy because of its accuracy, precision, speed of response and reliability. Infrared absorption spectroscopy capnometers quantify the subject's airway $CO_2$ gas exchange in real time without any airway perturbation or violation of sterility. Unfortunately, IR-based capnometry are technically complex and expensive compared to other common medical parameter measurements such as temperature, blood pressure, ECG, heart rate and pulse oximetry. Now that the use of capnometry has expanded outside the in-hospital environment to pre-hospital emergency care including non-intubated subject monitoring applications such as dentistry, pain management, conscious sedation, in-home use, etc., there is an increased awareness of the need for less expensive capnometry instruments. There is also a need for a low cost way of measuring a user's breathing rate and end-tidal $CO_2$ levels as part of a breathing therapy, such as those described in WO 2015/009792.

There are many other techniques for measuring gas exchange in a subject's breath. Among these include mass spectrometry, Raman scattering, photoacoustic, piezoelectric, paramagnetic and chemical based instruments. All of these techniques have specific tradeoffs with respect to their complexity, performance and cost. In examining the aspects of these tradeoffs, one technique stands alone as having potential for simplicity, meeting adequate performance criteria at considerably lower cost than other methods; the chemical based colorimetric technique.

Chemical based colorimetric techniques have been utilized in many other applications including qualitative human breath $CO_2$ detection. However, one of the challenges in using colorimetric techniques is its ability to achieve sufficient response time to capture rapidly changing $CO_2$ concentrations such as is found in a subject's ventilation pattern. Commercially available airway colorimetric products first appeared in the late 1980's, but could only give relative qualitative indications of $CO_2$ concentrations due to their slow response. In the 1990's, improvements to the indicator chemistry formulations were made to enhance the speed of response to breath-by-breath gas concentration variations. For example, in 1994 Dr. Andras Gedeon published test results of the response time of a qualitative colorimetric indicator compared with the response time of a quantitative IR spectroscopy-based capnometer showing significant similar breath-by-breath response times. Details regarding these test results are described in the paper "A New Colorimetric Breath Indicator (Colibri)" published in Anesthesia (1994) volume 49, pages 798-803, which is herein incorporated by reference in its entirety. Since then, Dr. Gedeon and others have continued to develop and manufacture qualitative colorimetric indicators primarily for use with intubation verification.

### SUMMARY OF THE DISCLOSURE

WO 2015/009792 discloses quantitative colorimetric indicators that interrogate the color change of a chemical colorimetric indicator to quantify carbon dioxide concentrations. The use of some chemical colorimetric indicators can present additional challenges, such as batch to batch differences in the chemical colorimetric indicator and changes to the colorimetric indicator after manufacture and prior to use. The colorimetric indicator can be sensitive to environmental factors. Contaminants and age can decrease the responsiveness and in turn accuracy of the quantitative colorimetric indicator. The colorimetric indicator can be replaced periodically or when the performance quality is decreased. Improved methods and apparatuses are provided herein for replacing the colorimetric indicator in the quantitative colorimetric indicator. Each colorimetric indicator may have slightly different responsiveness to carbon dioxide. Each colorimetric indicator can be characterized prior to packaging and use. Calibration of the colorimetric indicator after removal from the packaging and prior to use can present challenges to the user. Improved methods and apparatuses for calibrating colorimetric indicators are desired and disclosed herein. These characterization and calibration techniques can improve reliability, accuracy and ease of use of the quantitative colorimetric indicator.

The present disclosure relates to quantitative colorimetric systems and methods for using the same. The methods can include characterization and calibration methods for the colorimetric indicator. The quantitative colorimetric systems can be used to provide a user with a breathing therapy. Cartridges are also disclosed including a chemical colorimetric indicator along with a gas container having a reference gas.

In general, in one example, a method of calibrating a quantitative colorimetric measurement system including humidifying a chemical colorimetric indicator; exposing the chemical colorimetric indicator to a first gas; measuring a first color of the chemical colorimetric indicator based on the exposure to the first gas; exposing the chemical colorimetric indicator to a second gas having a different $CO_2$ concentration than the first gas; measuring a second color of the chemical colorimetric indicator based on the exposure to the second gas; and deriving a span $CO_2$ calibration based on the difference between the first color of the chemical colorimetric indicator and the second color of the chemical colorimetric indicator.

This and other examples can include one or more of the following features. Humidifying the chemical colorimetric indicator can include contacting the chemical colorimetric indicator with air exhaled by a user of the quantitative colorimetric $CO_2$ measurement system. The method can further include humidifying the chemical colorimetric indicator after measuring the first color of the chemical colorimetric indicator and prior to exposing the chemical colorimetric indicator to the second gas. Humidifying the chemical colorimetric indicator can include contacting the chemical colorimetric indicator with air exhaled by a user of the quantitative colorimetric measurement system. Humidifying the chemical colorimetric indicator can include contacting the chemical colorimetric indicator with a humidity source. The quantitative colorimetric measurement system can include a first removable cartridge which can include the chemical colorimetric indicator and a second removable cartridge including the humidity source, which can further include humidifying the chemical colorimetric indicator by passing ambient air through the humidity source into contact with the chemical colorimetric indicator. The first removable cartridge can further include a humidity-moisture exchanger (HME). Humidifying can include passing ambient air through the humidity source, HME, and the colorimetric indicator. Humidifying the chemical colorimetric indicator can include humidifying the chemical colorimetric indicator to a relative humidity of greater than about 60%. Humidifying the chemical colorimetric indicator can include humidifying the chemical colorimetric indicator to a relative humidity of greater than about 90%. The method can include humidifying the chemical colorimetric indicator; exposing the chemical colorimetric indicator to the first gas; measuring the first color of the chemical colorimetric indicator based on the exposure to the first gas; exposing the chemical colorimetric indicator to the second gas having a different $CO_2$ concentration than the first gas; measuring the second color of the chemical colorimetric indicator based on the exposure to the second gas; and deriving the span $CO_2$ calibration based on the difference between the first color of the chemical colorimetric indicator and the second color of the chemical colorimetric indicator can be performed in about 5 minutes or less. The method can further include applying the span calibration to a measurement of a color of the chemical colorimetric indicator exposed to a breath sample. Exposing the chemical colorimetric indicator to a second gas can include exposing the indicator to a sealed container filled with a reference sample having a known carbon dioxide concentration. The known carbon dioxide concentration can be from about 4% to about 7% carbon dioxide. The sealed container filled with the reference sample having the known carbon dioxide concentration can have a known humidity content. Exposing the indicator to the first gas can include exposing the chemical colorimetric indicator to a sealed container filled with a reference sample having a known carbon dioxide concentration. The sealed container filled with the reference sample having the known carbon dioxide concentration can have a known humidity content. Humidifying the chemical colorimetric indicator can include contacting the first gas with a humidification source. Exposing the indicator to the first gas can include exposing the chemical colorimetric indicator to ambient air. The method can further include engaging a first cartridge with the quantitative colorimetric measurement system, the first cartridge can contain the chemical colorimetric indicator. The method can further include engaging a second cartridge with the quantitative colorimetric measurement system, the second cartridge can contain the reference gas and a humidity source. A breathing therapy method can include calibrating a quantitative colorimetric measurement system using any of the above methods and can use the quantitative colorimetric measurement system for a breathing therapy for up to seven days. A breathing therapy method can include calibrating a quantitative colorimetric measurement system using any of the above methods and can use the quantitative colorimetric measurement system for a breathing therapy greater than 28 days. The method can further include removing the second cartridge containing the reference gas and the humidity source and engaging a fresh second cartridge containing a second reference gas and a second humidity source with the quantitative colorimetric measurement system. The method can further include using the quantitative colorimetric measurement system with the fresh second cartridge for a breathing therapy for up to seven days. The method can further include replacing the chemical colorimetric indicator with a second chemical colorimetric indicator after about 5-7 days. The method can further include humidifying the second chemical colorimetric indicator; exposing the second chemical colorimetric indicator to a first gas; measuring a first color of the second chemical colorimetric indicator based on the exposure to the first gas; exposing the second chemical colorimetric indicator to a second gas; measuring a second color of the second chemical colorimetric indicator based on the exposure to the second gas having a different $CO_2$ concentration than the first gas; and deriving a span calibration based on the difference between the first color of the second chemical colorimetric indicator and the second color of the second chemical colorimetric indicator. Humidifying the second chemical colorimetric indicator can include contacting the second chemical colorimetric indicator with a humidity source. The method can further include humidifying the second chemical colorimetric indicator by passing ambient air through the humidity source into contact with the chemical colorimetric indicator. Humidifying the second chemical colorimetric indicator can include humidifying the second chemical colorimetric indicator to a relative humidity of greater than about 60%. The first gas can have a

concentration of $CO_2$ of about 0% to about 2%. The method can further include verifying the humidity of the chemical colorimetric indicator after humidifying the chemical colorimetric measurement system. Verifying the humidity of the chemical colorimetric indicator can include measuring a color of a humidity sensor. The method can further include determining if the color of the humidity sensor corresponds to a humidity above a threshold humidity level. The method can further include after verifying the humidity of the chemical colorimetric indicator, measuring the first color of the chemical colorimetric indicator based on the exposure to the first gas. The method can further include measuring a temperature of the chemical colorimetric indicator when measuring the first color of the chemical colorimetric indicator. The method can further include applying the temperature of the chemical colorimetric indicator to the measured first color of the chemical colorimetric indicator when measuring the first color of the chemical colorimetric indicator. The method can further include measuring a humidity of the chemical colorimetric indicator when measuring the first color of the chemical colorimetric indicator. The method can further include determining an adjusted measurement signal corresponding to the first color of the chemical colorimetric indicator and the measured humidity of the chemical colorimetric indicator. The method can further include receiving an exhaled breath sample from a user; contacting the chemical colorimetric indicator with the exhaled breath sample; measuring a temperature of the chemical colorimetric indicator; measuring a humidity of the chemical colorimetric indicator; measuring a color of the chemical colorimetric indicator based on the exposure to the exhaled breath sample; obtaining a voltage measurement corresponding to the color of the colorimetric indicator and the humidity of the chemical colorimetric indicator; and determining a quantitative value of the $CO_2$ in the exhaled breath sample from the humidity of the chemical colorimetric indicator, the temperature of the chemical colorimetric indicator, and the voltage measurement. The method can further include comparing the span calibration to a factory characterization data for the chemical colorimetric indicator. The method can further include adjusting a calibration curve for the colorimetric indicator based on the comparison of the factory characterization data and the calibration data. The quantitative colorimetric measurement system can include a light source to determine the color of the chemical colorimetric indicator, and can further include adjusting properties of the light source to align the span calibration with the factory characterization data. Adjusting the properties of the light source can include modifying an electrical current supplied to the light source.

In general, in one example, a method of providing a breathing therapy including receiving an exhaled breath sample from a user; contacting the chemical colorimetric indicator with the exhaled breath sample; measuring a temperature of the chemical colorimetric indicator; measuring a humidity of the chemical colorimetric indicator; measuring a color of the chemical colorimetric indicator based on the exposure to the exhaled breath sample; obtaining a voltage measurement corresponding to the color of the colorimetric indicator and the humidity of the chemical colorimetric indicator; and determining a quantitative value of the $CO_2$ in the exhaled breath sample from the humidity of the chemical colorimetric indicator, the temperature of the chemical colorimetric indicator, and the voltage measurement.

This and other examples can include one or more of the following features. The method can further include outputting a set of visual and/or audio cues from the quantitative colorimetric system with instructions for the user to adjust their breathing pattern to coincide with the cues to thereby modify the user's exhaled $CO_2$ levels. The method can further include providing the set of visual cues including a visual indication of a respiration rate relative to a target respiration rate. The visual indication of the respiration rate can include a circular pattern with an increasing and decreasing diameter corresponding to the user's exhaled $CO_2$ level. The visual indication of the target respiration rate can include a circular target pattern. The method can further include changing a diameter of the circular target pattern of the target respiration rate based on a difference between the user's exhaled $CO_2$ levels and a target user's exhaled $CO_2$ levels. The method can further include changing a location of a target line to vary a distance between the target line and a reference point based on a difference between the user's exhaled $CO_2$ levels and a target user's exhaled $CO_2$ levels. The breathing pattern can include the exhaled $CO_2$ level and respiration rate. The method can further include displaying the user's measured $CO_2$ levels to provide visual feedback during treatment. The method can further include the therapy directing the user's end-tidal $CO_2$ levels to a level between about 37 mmHg and 43 mmHg. The method can further include treating post-traumatic stress disorder (PTSD), panic disorder, anxiety, asthma, hypertension, obsessive-compulsive disorder, social phobia, depression, apnea, migraines, or epilepsy by training the user to modify their exhaled $CO_2$ levels.

In general, in one example, an apparatus including a cartridge including a chemical colorimetric indicator and a humidity moisture exchanger, the cartridge configured to removably engage with a quantitative colorimetric measurement system.

This and other examples can include one or more of the following features. The apparatus can further include a sealed container including a reference gas including a known concentration of carbon dioxide. The known carbon dioxide concentration can be from about 4% to about 7% carbon dioxide. The sealed container can be configured for a single use. The sealed container can be configured to be resealable. The sealed container can be integral with the cartridge. The sealed container can be separate from the cartridge. The sealed container can contain a known humidity content. The apparatus can further include a second sealed container having a second known concentration of carbon dioxide. The second known concentration of carbon dioxide can be less than about 2% carbon dioxide. The second sealed container can contain a known humidity content. The apparatus can further include a humidification module configured to humidify an incoming gas prior to the gas contacting the chemical colorimetric indicator. The humidification module can include a humidity reservoir. The humidification module can include a wet filter. The humidification module can include a water reservoir. The sealed container can be contained in a second removable cartridge configured to removably engage with a quantitative colorimetric measurement system. The apparatus can further include a humidity source in the second removable cartridge. The cartridge can further include a humidity sensor. The apparatus can further include the quantitative colorimetric measurement system. The quantitative colorimetric measurement system can further include a humidity sensor. The quantitative colorimetric measurement system can include an electro-optical sensor assembly including one or more light sources and a light detection material configured to detect light reflected off of the chemical colorimetric indicator by the light source. The electro-optical sensor assembly can further be configured to generate an electrical signal based on the light detected by the light detection material. The apparatus can further include a processor in communication with the electro-optical sensor assembly. The processor can be configured to receive the electrical signals generated by the electro-optical sensor assembly. The processor can utilize the signals to compute the quantity of carbon dioxide exposed to the chemical colorimetric indicator. The chemical colorimetric indicator can be adapted to change color in response to exposure to a quantity of carbon dioxide gas. The electro-optical assembly can be configured to detect light reflected off of the humidity sensor. The humidity sensor can be adapted to change color in response to exposure to humidity. The humidity sensor can be downstream of the chemical colorimetric indicator.

According to the invention, a quantitative colorimetric measurement system including an electro-optical sensor assembly including one or more light sources and a light detection material configured to detect light reflected off of a chemical colorimetric indicator by the light source, the electro-optical sensor assembly configured to generate an electrical signal based on the light detected by the light detection material; a humidity sensor adapted to measure a humidity of the chemical colorimetric indicator; a temperature sensor adapted to measure a temperature of the chemical colorimetric indicator; a processor configured to determine a quantitative value of $CO_2$ detected by the chemical colorimetric indicator from the humidity of the chemical colorimetric indicator, the temperature of the chemical colorimetric indicator, and a voltage measurement corresponding to the electrical signal based on the light detected by the light detection material; and a removable cartridge including the chemical colorimetric indicator and a humidity moisture exchanger, the cartridge configured to removably engage with the quantitative colorimetric measurement system.

This and other embodiments can include one or more of the following features. The system can further include a second removable cartridge including a humidity source and a reference gas having a known concentration of carbon dioxide, the second cartridge can be configured to removably engage with the quantitative colorimetric measurement system. In general, in one embodiment, a breathing therapy method including receiving at least a portion of a user's exhaled air in a gas inlet of a quantitative colorimetric measurement system; measuring the humidity of a humidity sensor within the quantitative colorimetric measurement system that is exposed to the user's exhaled air; confirming that the humidity of the humidity sensor is above a threshold humidity; and measuring a user's end-tidal $CO_2$ levels with the quantitative colorimetric measurement system based on a color change resulting from exposure of the system to the user's exhaled air.

This and other embodiments can include one or more of the following features. The method can further include outputting a set of visual and/or audio cues from the quantitative colorimetric system with instructions for the user to adjust their breathing pattern to coincide with the cues to thereby modify the user's exhaled $CO_2$ levels. The breathing pattern can include the exhaled $CO_2$ level and respiration rate. The method can further include displaying the user's measured $CO_2$ levels to provide visual feedback during treatment. The method can further include displaying the user's breathing rate to provide visual feedback during treatment. The method can further include the therapy directing the user's end-tidal $CO_2$ levels to a level between about 37 mmHg and 43 mmHg. The method can further include treating post-traumatic stress disorder (PTSD), panic disorder, anxiety, asthma, hypertension, obsessive-compulsive disorder, social phobia, depression, apnea, migraines, or epilepsy by training the user to modify their exhaled $CO_2$ levels. The method can further include measuring a temperature of a chemical colorimetric indicator of the quantitative colorimetric measurement system when measuring the user's end-tidal $CO_2$ levels. The method can further include applying a temperature correction to measured user's end-tidal $CO_2$ levels based on the temperature of the chemical colorimetric indicator when measuring the user's end-tidal $CO_2$ levels.

In general, in one example, a method for characterizing a chemical colorimetric indicator including contacting the chemical colorimetric indicator sequentially with a plurality of gases having a plurality of different concentrations of carbon dioxide; measuring a color of the chemical colorimetric indicator based on an exposure to each of the plurality of gases by reflecting a first light source having a first wavelength off of the chemical colorimetric indicator and detecting a first light reflected from the colorimetric indicator to generate a plurality of measurement signals; determining a characterization for the chemical colorimetric indicator by analyzing the plurality of measurement signals corresponding to the plurality of different concentrations of carbon dioxide; and calculating a calibration curve for the chemical colorimetric indicator based on the characterization for the chemical colorimetric indicator.

This and other examples can include one or more of the following features. The calibration curve can be a polynomial equation that can be a function of the measurement signal. The calibration curve can be a polynomial equation that can be a function of the measurement signal and a temperature of the chemical colorimetric indicator. The polynomial equation can include: $CO2(V) = [A' * V^3] + [B' * V^2] + [C' * V] + D'$. The polynomial equation can include: $CO2(V,T) = [A'(T) * V^3] + [B'(T) * V^2] + [C'(T) * V] + D'(T)$ with

$$A'(T) = a_1 * T^2 + b_1 * T + c_1$$

$$B'(T) = a_2 * T^2 + b_2 * T + c_2$$

$$C'(T) = a_3 * T^2 + b_3 * T + c_3$$

$$D'(T) = a_4 * T^2 + b_4 * T + c_4.$$

Calculating the calibration curve can include calculating a plurality of coefficients for the calibration curve. The plurality of $CO_2$ concentrations can be from about 0% to about 8%. The method can further include measuring the color of the chemical colorimetric indicator by reflecting a plurality of light sources having a plurality of wavelengths off of the chemical colorimetric indicator and detecting a plurality of reflected light to generate a plurality of measurement signals. The method can further include measuring the color of the chemical colorimetric indicator at a plurality of different temperatures. The plurality of temperatures can be between about 15°C and 31°C. The method can further include deriving coefficients for the temperature sensitive functions of the calibration curve. The method can further include measuring the color of the chemical colorimetric indicator at a plurality of different humidity levels. The method can further include deriving coefficients for a humidity sensitive function for the measurement signal of the chemical colorimetric indicator. The coefficients for the humidity sensitive function can include p, q, and r from the following equation with RH referring to a relative humidity: $F_{RH} = \left( \frac{1}{p*RH^2+q*RH+r} \right)$. The method can further include recording the calibration curve for the chemical colorimetric indicator. The method can further include packaging the chemical colorimetric indicator. The method can further include encoding the calibration curve with packaging.

In general, in one example, a method of calibrating a chemical colorimetric indicator including exposing the chemical colorimetric indicator to a first gas having a first concentration of carbon dioxide; measuring a color of the chemical colorimetric indicator based on the exposure to the first gas by reflecting a first light source having a first wavelength off of the chemical colorimetric indicator and detecting a first light reflected from the colorimetric indicator to generate a first measurement signal; exposing the chemical colorimetric indicator to a second gas having a second concentration of carbon dioxide; measuring the color of the chemical colorimetric indicator based on the exposure to the second gas by reflecting the first light source having the first wavelength off of the chemical colorimetric indicator and detecting the second light reflected from the colorimetric indicator to generate a second measurement signal; determining a span $CO_2$ calibration based on the difference between the first measurement signal and the second measurement signals; and comparing the span calibration to a factory characterization data for the chemical colorimetric indicator.

This and other examples can include one or more of the following features. The method can further include adjusting a calibration curve for the colorimetric indicator based on the comparison of the factory characterization data and the calibration data. The method can further include adjusting the first light source properties to align the span calibration with the factory characterization data. The method can further include optimizing the first light source properties to align the span calibration with the factory characterization data. Optimizing can include adjusting the first light source properties to match the first measurement signal and second measurement signal to the characterization data. The method can further include removing the chemical colorimetric indicator from a package containing the colorimetric indicator. The package can include the factory characterization data for the chemical colorimetric indicator. The method can further include engaging the chemical colorimetric indicator with a quantitative colorimetric measurement system. The method can further include providing the factory characterization data for the chemical colorimetric indicator to the quantitative colorimetric measurement system. The method can further include humidifying a chemical colorimetric indicator prior to exposing the chemical colorimetric indicator to the first gas. The method can further include monitoring a user's breathing using the quantitative colorimetric measurement system. The first measurement signal and plurality of second measurement signals can include a voltage measurement detected by one or more photodiodes from the light reflected from the chemical colorimetric indicator.

In general, in one example, a kit including a chemical colorimetric indicator configured to be used with a quantitative carbon dioxide measurement system; and characterization data for the chemical colorimetric indicator.

This and other embodiments can include one or more of the following features. The characterization data can include coefficients for a calibration curve. The calibration curve can be a polynomial equation and the coefficients correspond to the polynomial equation. The coefficients can include coefficients for a temperature sensitive equation. The calibration curve can be a function of a voltage measured by the quantitative carbon dioxide measurement system. The kit can further include a sealed packaging material containing the chemical colorimetric indicator. The packaging material can be moisture resistant and light resistant. The characterization data can be encoded on a packaging of the chemical colorimetric indicator in a machine readable format. The polynomial equation can include:

$$CO2(V) = [A' * V^3] + [B' * V^2] + [C' * V] + D'.$$

The polynomial equation can include:

$$CO2(V,T) = [A'(T) * V^3] + [B'(T) * V^2] + [C'(T) * V] + D'(T)$$

with

$$A'(T) = a_1 * T^2 + b_1 * T + c_1$$

$$B'(T) = a_2 * T^2 + b_2 * T + c_2$$

$$C'(T) = a_3 * T^2 + b_3 * T + c_3$$

$$D'(T) = a_4 * T^2 + b_4 * T + c_4.$$

The coefficients can include coefficients for a humidity sensitive equation. The coefficients for the humidity sensitive equation can include p, q, and r from the following equation with RH referring to a relative humidity: $F_{RH} = \left(\frac{1}{p*RH^2+q*RH+r}\right)$. The chemical colorimetric indicator can be part of a removable cartridge configured to removably engage with the quantitative carbon dioxide measurement system. The kit can further include a humidity moisture exchanger.

In general, in one example, a method of calibrating a quantitative colorimetric measurement system including instructing a user of the quantitative colorimetric measurement system to provide a breath sample to a chemical colorimetric indicator to humidify the chemical colorimetric indicator; verifying that the chemical colorimetric indicator has been humidified; instructing the user to expose the chemical colorimetric indicator to a first ambient gas; measuring a first color of the chemical colorimetric indicator based on the exposure to the first gas; instructing the user to expose the chemical colorimetric indicator to a reference gas; measuring a second color of the chemical colorimetric indicator based on the exposure to the reference gas; and deriving a span calibration based on the difference between the first color of the chemical colorimetric indicator and the second color of the chemical colorimetric indicator.

This and other examples can include one or more of the following features. The first gas can have a concentration of $CO_2$ of about 0% to about 2%. The reference gas can have a known carbon dioxide concentration of from about 4% to about 7% carbon dioxide. Verifying the humidity of the chemical colorimetric indicator can include measuring a color of a humidity sensor. The method can further include determining if the color of the humidity sensor corresponds to a humidity above a threshold humidity level. The method can further include after verifying the humidity of the chemical colorimetric indicator, instructing the user to expose the chemical colorimetric indicator to a first ambient gas. The method can further include measuring a temperature of the chemical colorimetric indicator when measuring the first color of the chemical colorimetric indicator. The method can further include applying the temperature of the chemical colorimetric indicator to the measured first color of the chemical colorimetric indicator when measuring the first color of the chemical colorimetric indicator. The chemical colorimetric indicator can be adapted to change color in response to exposure to a quantity of carbon dioxide gas. The method can further include comparing the span $CO_2$ calibration to a characterization data provided with the chemical colorimetric indicator. The method can further include adjusting a calibration of the chemical colorimetric indicator based on the comparison of the span $CO_2$ to the characterization data provided with the chemical colorimetric indicator. The method can further include adjusting one or more optical properties of the quantitative colorimetric measurement system to correlate the span $CO_2$ calibration to the characterization data. Adjusting one or more optical properties can include adjusting one or more light emitting diodes (LEDs) of the quantitative colorimetric measurement system. A system in accordance with the invention is defined in claim 1. Methods in accordance with the invention are defined in claims 4 and 9.

**BRIEF DESCRIPTION OF THE DRAWINGS**

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:

FIG. 1 illustrates a quantitative colorimetric gas detector in accordance with some embodiments.
FIG. 2 illustrates a quantitative colorimetric gas detector in accordance with some embodiments.
FIGS. 3A-3B illustrate process flow charts in accordance with some embodiments.
FIG. 4 illustrates a schematic diagram of a quantitative colorimetric measurement system in accordance with some embodiments.
FIG. 5 is a flowchart illustrating a method for calibrating a quantitative colorimetric measurement system in accordance with some embodiments.
FIG. 6 is a flowchart illustrating a method for calibrating a quantitative colorimetric measurement system in accordance with some embodiments.
FIG. 7A is a flowchart illustrating a method for characterizing a chemical colorimetric indicator in accordance with some embodiments.
FIG. 7B is a flowchart illustrating a method for calibrating a chemical colorimetric indicator in accordance with some embodiments.
FIG. 8 illustrates a recorded voltage signal versus time for a chemical colorimetric indicator in accordance with some embodiments.
FIG. 9A illustrates a graph of voltage versus CO2 at various temperatures. FIG. 9B illustrates a graph of CO2 versus voltage at various temperatures. FIG. 9C illustrates a graph of coefficients versus temperature for the CO2 to voltage data of FIG. 9A. FIG. 9D illustrates a graph of coefficients versus temperature for the voltage to CO2 of FIG. 9B. FIG. 9E illustrates corresponding fit coefficients in accordance with some embodiments.
FIG. 10A is a flowchart illustrating a method for a quantitative colorimetric measurement system providing a breathing therapy in accordance with some embodiments.
FIG. 10B is a flowchart illustrating a method for a quantitative colorimetric measurement system providing a breathing therapy in accordance with some embodiments.
FIG. 11 illustrates a graphical representation of end-tidal CO2 and breathing rate in accordance with some embodiments.
FIGS. 12A-12B illustrate a quantitative colorimetric measurement system including a removable cartridge with a chemical colorimetric indicator in accordance with some embodiments.
FIG. 13 illustrates a block diagram of quantitative colorimetric measurement system with a removable cartridge with a chemical colorimetric indicator in accordance with some embodiments.
FIG. 14 illustrates is an isometric illustration of the components of a removable cartridge for use with a quantitative colorimetric measurement system in accordance with some embodiments.
FIGS. 15A-15B illustrate a quantitative colorimetric measurement system including a removable cartridge and calibration gas with a chemical colorimetric indicator in accordance with some embodiments.
FIG. 16 is an isometric illustration of the components of a removable cartridge for use with a quantitative colorimetric measurement system in accordance with some embodiments.
FIGS. 17A-17C illustrate removable cartridges including a chemical colorimetric indicator in accordance with some embodiments.
FIG. 18 illustrates aspects of quantitative colorimetric measurement systems in accordance with some embodiments.
FIG. 19 illustrates various humidity sensors in accordance with some embodiments.
FIG. 20 is an isometric illustration of the components of a removable cartridge for use with a quantitative colorimetric measurement system including a humidity sensor in accordance with some embodiments.
FIG. 21 is a schematic diagram of a quantitative colorimetric measurement system in accordance with some embodiments.

FIG. 22 illustrates a quantitative colorimetric measurement system with a computer display device in accordance with some embodiments.

FIG. 23 illustrates a quantitative colorimetric measurement system including a cannula case in accordance with some embodiments.

FIG. 24 illustrates a quantitative colorimetric measurement system including a first removable cartridge containing carbon dioxide and a second cartridge containing a chemical colorimetric indicator in accordance with some embodiments.

FIG. 25 illustrates an embodiment of a user interface displayed on a handheld computing device.

FIG. 26 illustrates an embodiment of a user interface.

FIG. 27 illustrates an exploded view of a removable cartridge including a chemical colorimetric indicator in accordance with some embodiments.

FIG. 28 illustrates a view of a removable cartridge with a carbon dioxide reference gas in accordance with some embodiments.

FIG. 29 illustrates a quantitative colorimetric measurement system including a first removable cartridge containing carbon dioxide and a second cartridge containing a chemical colorimetric indicator in accordance with some embodiments.

FIG. 30 illustrates a quantitative colorimetric measurement system in accordance with some embodiments.

## DETAILED DESCRIPTION

Apparatuses, methods, and kits are disclosed herein for use in quantitative colorimetric measurement systems. The methods can include improved methods for characterizing and calibrating a chemical colorimetric indicator used in the quantitative colorimetric measurement system.

The chemical colorimetric indicator can be characterized in a laboratory setting prior to packaging. However, the chemical colorimetric indicator can change after packaging and through exposure to environmental elements. Consequently, it is desirable to further calibrate the chemical colorimetric indicator prior to or at the time of use to improve the accuracy and reliability of the quantitative colorimetric indicator.

Calibrating the chemical colorimetric indicator outside of the laboratory, such as at home, can be challenging. Typically, laboratories have a clean environment, access to reference gases, and a technician that has experience with performing the calibration. A home environment usually does not have all of these conditions. It is desirable to provide an easy and reliable calibration process that can be used by a patient or subject at home prior to receiving a breathing therapy. It is also desirable for the calibration to be automatic or to require few user steps or intervention. In some embodiments the calibration of the chemical colorimetric indicator can happen without user intervention or input. The calibration of the chemical colorimetric indicator can also take about 5 minutes or less.

The calibration steps can include sampling room air/ambient air to approximate a reference gas having a carbon dioxide concentration close to zero along with sampling a reference gas having a known carbon dioxide concentration. The carbon dioxide content of the room air can be estimated. For example, a carbon dioxide content of 0% to about 0.10% can be assumed for the room air. In some embodiments the carbon dioxide content of the room air is assumed to be about 0.07%. The reference gas can be provided with the chemical colorimetric indicator. The reference gas can have a known concentration around the desired upper range for analysis, such as a concentration between about 4% to 6% carbon dioxide. The quantitative colorimetric indicator can reflect light off of the colorimetric indicator and measure the reflected light to produce a measurement signal. The measurement signals can be converted to a corresponding carbon dioxide level using the characterization and calibration data for the specific chemical colorimetric indicator.

Another challenge with properly characterizing and calibrating a chemical colorimetric indicator is that the chemical colorimetric indicator or film typically requires a threshold level of humidification in order to accurately and properly respond to the concentration of the carbon dioxide. The film can be humidified controllably in a laboratory setting with the proper materials and procedures; however, this can be challenging in an environment outside of the laboratory during calibration prior to use. One humidification source for calibration prior to use is the exhaled breath of the patient or subject using the quantitative colorimetric indicator. The chemical colorimetric indicator can be humidified prior to calibration by prompting the user/subject to exhale into the device or by sampling the exhaled breath. The chemical colorimetric indicator can then be calibrated using the room air and a reference gas after humidification of the chemical colorimetric indicator.

The humidification can result in surface adsorption of water on the surface of the chemical colorimetric indicator. Once the adsorption of water has exceeded a threshold amount the chemical colorimetric indicator can be used. The adsorption of water on the surface of the chemical colorimetric indicator is different than directly contacting the chemical colorimetric indicator with liquid phase water. Liquid saturation or liquid contacting the chemical colorimetric indicator can adversely change the properties of the chemical colorimetric indicator and change the chemical colorimetric indicator's response to carbon dioxide. Fully saturating the chemical colorimetric indicator with water can prevent the chemical colorimetric indicator from working or predictably responding to gas phase carbon dioxide.

In some embodiments the humidification threshold level is between about 10% and 100% humidification. In some embodiments the humidification threshold level is between about 40% and 100% humidification. In some embodiments the humidification threshold level is between about 60% and 100% humidification. In some embodiments the humidification threshold level is greater than about 40% humidification. In some embodiments the humidification threshold level is greater than about 60% humidification.

The measurement signal produced by the interrogation of the chemical colorimetric indicator can also vary with the humidity of the indicator. The humidity of the chemical colorimetric indicator or the environment adjacent to or surrounding the chemical colorimetric indicator can be measured with a humidity sensor or probe. A humidity compensation or correction can then be applied to the measurement signal corresponding to the color of the chemical colorimetric indicator.

The chemical colorimetric indicator can be humidified in other ways in some embodiments. For example, humidification of the colorimetric material can be done by a humidifier component, humidification module, humidity source, etc. In some embodiments the user can put water into the humidifier component. In some embodiments the humidifier component can be prepackaged with a moisture level. The user can then open the humidifier component and place the humidifier in the system prior to calibration. In some embodiments the humidity source can be provided in a removable cartridge. Ambient or room air can be pulled into the device and humidified with the humidity source from the removable cartridge. The humidified air can be used to humidify components of the systems, such as the chemical colorimetric indicator. The humidifier component can also be included as part of a cartridge also containing the calibration gas.

Step by step instructions can be provided to the subject using the quantitative colorimetric indicator to perform the calibration steps described herein.

In some embodiments the humidification of the chemical colorimetric indicator can be verified by interrogating a humidity sensor with the light source and measuring the reflected light. After verifying the humidification of the chemical colorimetric indicator the subject can perform a breathing therapy with the quantitative colorimetric indicator. Methods for providing a breathing therapy are disclosed in WO 2015/009792, which is incorporated by reference in its entirety. Any of the breathing therapy methods disclosed in WO 2015/009792 can be used with the devices and methods disclosed herein.

The apparatuses can include cartridges with the chemical colorimetric indicator that can be used with the quantitative colorimetric measurement system. The cartridges can removably engage with the housing of the quantitative colorimetric measurement system. After the user is done with the chemical colorimetric indicator the chemical colorimetric indicator needs to be replaced the cartridge can be removed and a fresh cartridge can be inserted into the quantitative colorimetric measurement system. The cartridge/chemical colorimetric indicator can be provided with a reference gas having a known concentration of carbon dioxide. The reference gas can be provided in a container integral with the cartridge or separate from the cartridge. The chemical colorimetric indicator can be calibrated after engaging the cartridge with the quantitative colorimetric measurement system.

The cartridges can also include characterization data determined during the characterization of the chemical colorimetric indicator at the laboratory prior to packaging. The quantitative colorimetric measurement system can read (e.g., via RFID, barcode, memory chip internal to the cartridge, etc.) the encoded calibration data for the chemical colorimetric indicator prior to or when the cartridge is engaged with the rest of the system. The encoded laboratory characterization data can be compared to the calibration data/curve determined during the calibration done by the user and adjustments made if necessary.

More specific examples of the methods and apparatuses disclosed herein are discussed below with reference to the Figures and examples. Figures describe aspects of quantitative colorimetric carbon dioxide measuring systems that can be used with the method and apparatuses disclosed herein.

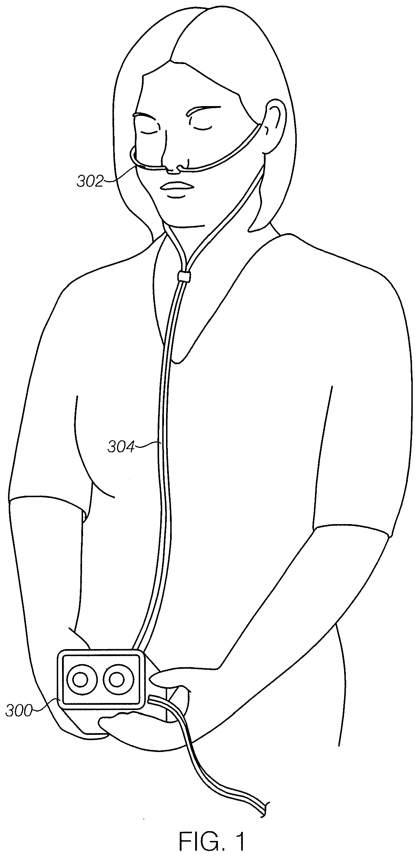

FIG. 1 illustrates an example of a patient using a quantitative colorimetric carbon dioxide measuring system 300 in some embodiments. Exhaled breath of the patient enters an inlet 302, illustrated as a nasal cannula, and flows through a conduit or cannula 304 and into the quantitative colorimetric carbon dioxide measuring system 300.

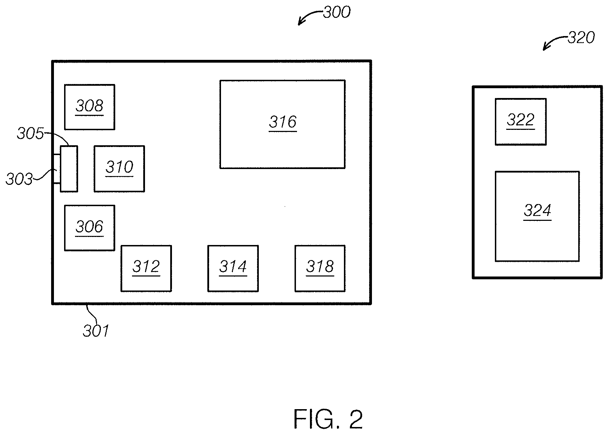

FIG. 2 illustrates a schematic of a quantitative colorimetric carbon dioxide measuring system 300 in accordance with some embodiments. The system 300 includes a conduit or inlet 303. The conduit or inlet 303 can be configured to receive or engage with a cannula, sample inlet tube, or other conduit such that the cannula, conduit, or inlet is configured to introduce a gas sample to the system 300. The system 300 can include a chemical colorimetric indicator 305 within a housing 301 of the system 300. The colorimetric indicator 305 can be part of a removable cartridge as disclosed herein. An electro-optical sensor 306 can be included to interrogate the colorimetric indicator 305. An optional temperature controller 308 can be provided to control the temperature of the colorimetric indicator and/or the temperature of the incoming gas sample. In some embodiments an optional temperature probe or sensor can be used to measure the temperature of the colorimetric indicator, incoming gas sample, and/or electro-optical sensor. In some optional embodiments the temperature of the colorimetric indicator can be measured while analyzing the sample gas. In some optional embodiments a temperature correction can be applied as described herein to adjust the measured signal to compensate for the temperature of the colorimetric indicator. In some embodiments the temperature is used to calculate the carbon dioxide content of the sample gas in combination with the measured signal corresponding to the color of the chemical colorimetric indicator.

A pump 310 can be included within the housing 301 to pump the incoming gas sample. In some embodiments the pump 310 can be located downstream of the colorimetric indicator to effectively pull the incoming gas sample passed the porous colorimetric indicator. In some embodiments the pump can be upstream of the colorimetric indicator to pump the gas sample passed the colorimetric indicator. The system 300 includes operating electronics 312. The operating electronics can control the system to perform various processing steps as described herein. In some embodiments the operating electronics receive the measurement signal from the electro-optical assembly and calculate properties associated the measurement signal. In some embodiments the operating electronics receive the measurement signal and send the measurement signal to a processor external to the system 300, with the external processor performing the calculations and analysis of the measurement signal. In some embodiments the system 300 includes a wireless transmitter 314 to transmit data to an external processor, such as a processor on a computer, tablet computer, or smartphone. The wireless transmitter can transmit data via Bluetooth or other wireless data transfer protocol. The system 300 can include a power supply 318 to power the components of the system 300.

In some embodiments the system 300 can include a display 316 with the housing 301. In some embodiments the display is external to the system. For example, the display data can be wirelessly transmitted to a device having a display, such as a computer, smartphone, tablet computer, flat screen monitor, television, etc. In some embodiments a tablet computer or smartphone 320 can be used with the system 300. The tablet computer 320 can include a processor 322 and display 324. In some embodiments the processor 322 can receive the measurement signal transmitted by the system 300 and analyze the measurement signal to determine properties associated with the measurement signal. In some embodiments the processor 322 is configured to receive data from the system 300 and display the data on the tablet computer 320 display 324. Decreasing the processing steps performed by the processor on board the system 300 can reduce the complexity and cost of the system 300.

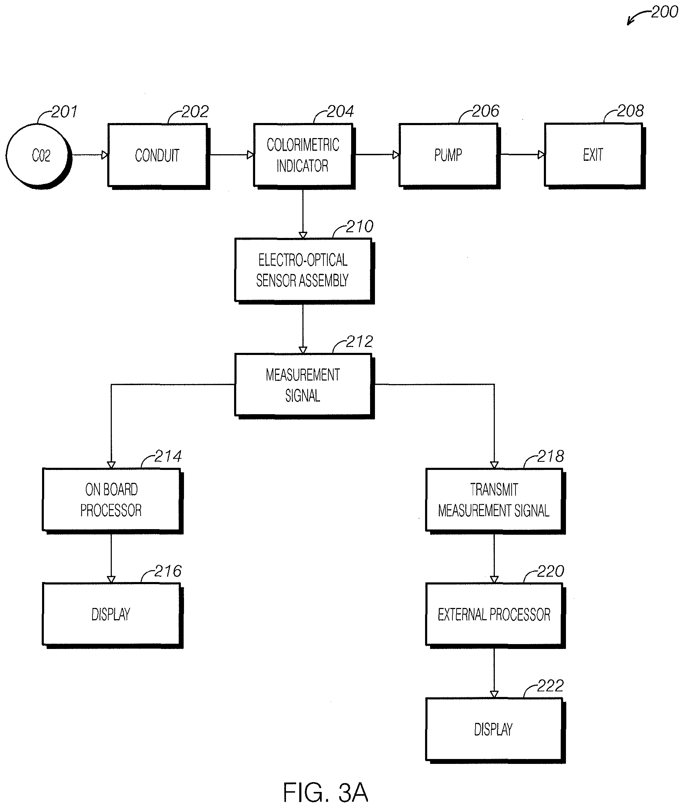

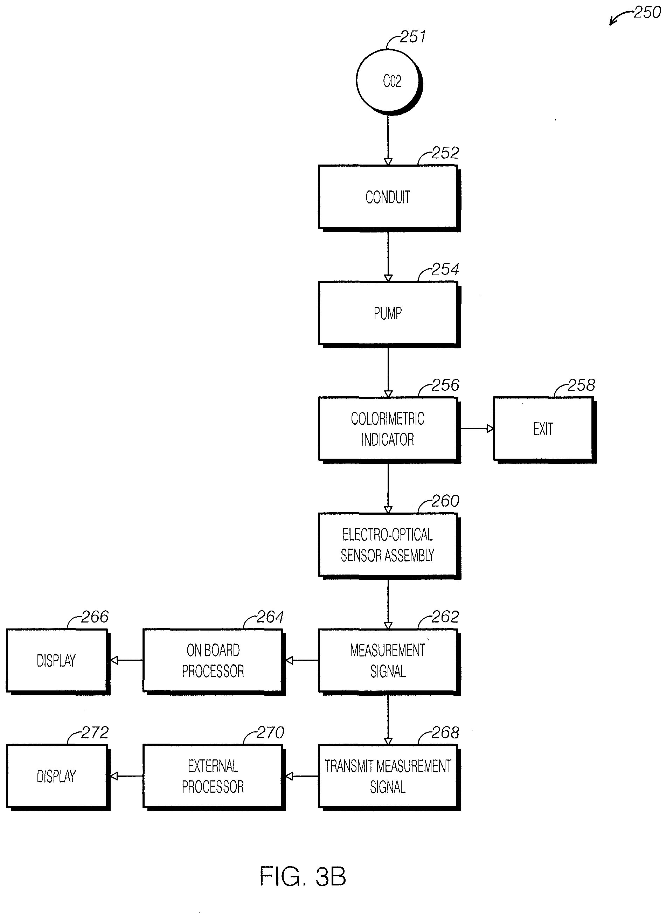

FIGS. 3A and 3B illustrate embodiments of flow charts 200, 250 for process flows. As shown in FIGS. 3A-3B a carbon dioxide sample 201, 251 enters a gas or fluid conduit 202, 252 and contacts the colorimetric indicator 204, 256. A pump 206, 254 can be used to pump the carbon dioxide into contact with the colorimetric indicator. The pump can be downstream of the colorimetric indicator (FIG. 3A) or upstream of the colorimetric indicator (FIG. 3B). The carbon dioxide can exit 208, 258 the system after contacting the colorimetric indicator 204, 256. The electro-optical sensor assembly 210, 260 interrogates the colorimetric indicator 204, 256 when the carbon dioxide stream contracts the colorimetric indicator 204, 256. The electro-optical sensor assembly 210, 260 outputs a measurement signal 212, 262 based on the interrogation of the colorimetric indicator 204, 256. The measurement signal 212, 262 can be sent to an onboard processor 214, 264 that analyzes the measurement signal 212, 262 to determine the amount of carbon dioxide contacting the colorimetric indicator 204, 256. As an alternative option the measurement signal 212, 262 can be transmitted to an external processor 220, 270 with the external processor determining the amount of carbon dioxide that contacts the colorimetric indicator 204, 256. Data associated with the interrogation of the colorimetric indicator can then be displayed 216, 222, 266, 272. The display can be onboard the device (216, 266), external to the device (222, 272), part of a tablet computer, smartphone, or computer in communication with the device.

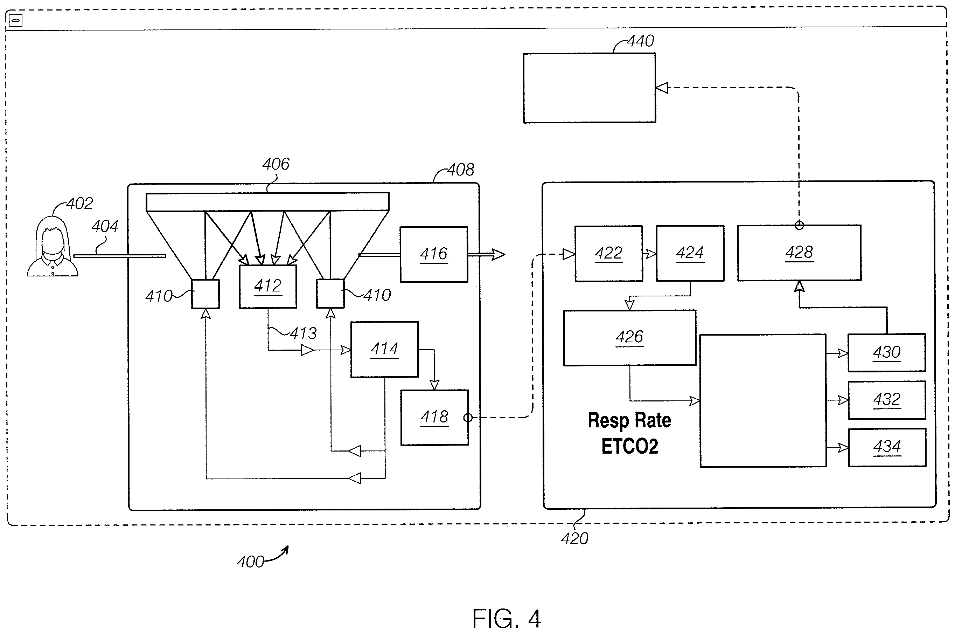

FIG. 4 illustrates a schematic diagram of a quantitative colorimetric measurement system 400 in accordance with some embodiments. FIG. 4 illustrates a patient 402 breathing into a sample tube 404 to provide expired air to the chemical colorimetric indicator 406 within the quantitative colorimetric capnometer 408. A plurality of LEDs 410 can interrogate the color of the chemical colorimetric indicator 406 by reflecting light off of the film 406 and measuring the reflected light with a photodetector 412. The exhaled breath sample can be cleared from the quantitative colorimetric capnometer 408 with a pump 416. The photodetector 412 can generate a measurement signal 413 based on the detected light that can be processed with a microprocessor 414 and wirelessly transmitted with a Bluetooth or other wireless data transmitter 418 to an external computing device, such as the illustrated tablet computer 420. The tablet computer 420 or hand held computing device can apply an algorithm to convert the observed reflected light into a quantitative carbon dioxide measurement. The tablet computer 420 can receive via wireless receiver/transmitter 422 the data from the capnometer 408. The tablet computer 420 can process the data with the processor 424 and apply an algorithm 426 to determine the respiratory rate and other characteristics of the exhaled breath sample. The tablet computer 420 can transmit data wirelessly to a remote server 440 with a wireless radio 428. The computer tablet 420 can also include a memory 430, display 432, and speaker 434. The illustrated capnometer shows two LEDs 410. In some embodiments two or more distinct spectra or wavelengths of light can be used to interrogate the chemical colorimetric indicator. In some embodiments a single LED can be used to interrogate the chemical colorimetric indicator at a single wavelength. In some embodiments three or more distinct spectra or wavelengths of light can be used to interrogate the chemical colorimetric indicator. In some embodiments four or more distinct spectra or wavelengths of light can be used to interrogate the chemical colorimetric indicator. The plurality of spectra or wavelengths can be alternately and sequentially provided to the chemical colorimetric indicator with the reflected signals detected and measured by the photodetector. In some embodiments the spectra or wavelengths are in the visible spectrum. Although the present disclosure generally focuses on interrogating the chemical colorimetric material with visible light and measuring the reflected light, in some embodiments the light can be in the invisible spectrum. In some embodiments the spectra or wavelength of light can be in the invisible spectrum. The terminology generally used herein describes wavelengths of light; however, a skilled artisan would appreciate that in embodiments that use invisible light or electromagnetic energy the wavelength terminology can be interchangeable with a spectra. The hand held computer can be used to provide any of the breathing therapies described herein to the patient. Data received from the capnometer and processed by the tablet computer can also be sent to a server for further analysis.

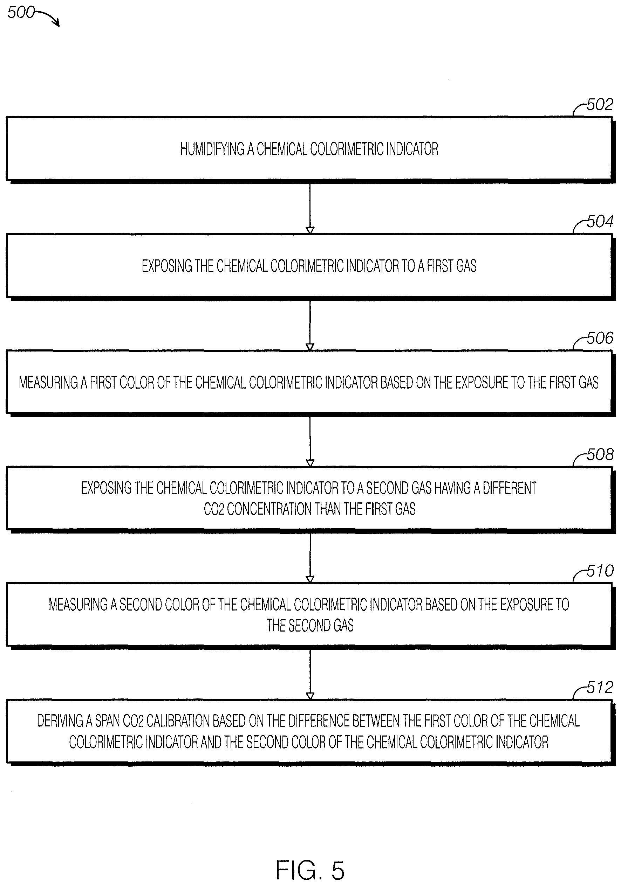

FIG. 5 illustrates a method 500 of calibrating a quantitative colorimetric $CO_2$ measurement system. The method includes humidifying a chemical colorimetric indicator 502, exposing the chemical colorimetric indicator to a first gas 504, measuring a first color of the chemical colorimetric indicator based on the exposure to the first gas 506, exposing the chemical colorimetric indicator to a second gas having a different $CO_2$ concentration than the first gas 508, measuring a second color of the chemical colorimetric indicator based on the exposure to the second gas 510, and deriving a span $CO_2$ calibration based on the difference between the first color of the chemical colorimetric indicator and the second color of the chemical colorimetric indicator 512.

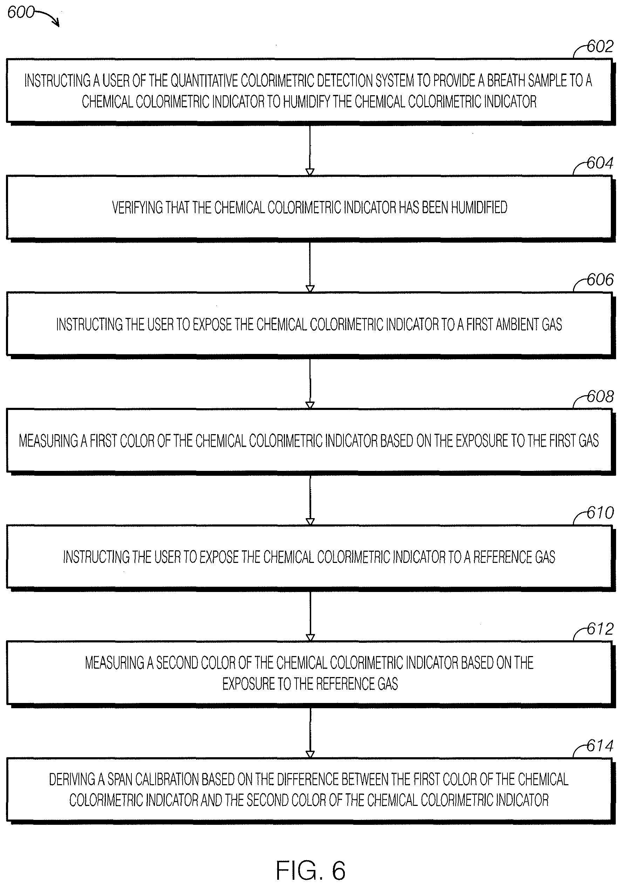

Methods are also disclosed herein for providing instructions to the user to perform steps of calibration. FIG. 6 is a flowchart illustrating a method 600 for calibrating a quantitative colorimetric measurement system comprising in accordance with some embodiments. The calibration includes instructing a user of the quantitative colorimetric measurement system to provide an exhaled breath sample to a chemical colorimetric indicator to humidify the chemical colorimetric indicator 602, verifying that the chemical colorimetric indicator has been humidified 604, instructing the user to expose the chemical colorimetric indicator to a first ambient gas 606, measuring a first color of the chemical colorimetric indicator based on the exposure to the first gas 608, instructing the user to expose the chemical colorimetric indicator to a reference gas 610, measuring a second color of the chemical colorimetric indicator based on the exposure to the reference gas 612, and deriving a span calibration based on the difference between the first color of the chemical colorimetric indicator and the second color of the chemical colorimetric indicator 614. The span calibration information may be obtained prior to first use of the colorimetric indicator and may optionally be used together with colorimetric indicator characterization information derived earlier, as described further below.

Humidifying the chemical colorimetric indicator can be done by providing a water or moisture source to the quantitative colorimetric measurement system. The water or moisture source can be used to humidify the chemical colorimetric indicator or film above a threshold humidification level. In some embodiments humidifying the chemical colorimetric indicator can include contacting the chemical colorimetric indicator with air exhaled by a user of the quantitative colorimetric measurement system. The air exhaled by the user of the quantitative colorimetric measurement system has a humidity level that can quickly and easily humidify the chemical colorimetric indicator. Humidifying can include contacting the chemical colorimetric material with air exhaled by the user for a set amount of time. For example, the chemical colorimetric material can be humidified after contacting it with exhaled air for several minutes. In some embodiments humidifying includes contacting the chemical colorimetric material with exhaled air for greater than about one minute. In some embodiments humidifying includes contacting the chemical colorimetric material with exhaled air for greater than about two minutes. In some embodiments humidifying includes contacting the chemical colorimetric material with exhaled air for about two minutes to about four minutes. In some embodiments humidifying includes contacting the chemical colorimetric material with exhaled air for greater than about four minutes.

In some embodiments water or moisture can be provided to the quantitative colorimetric measurement system to humidify the chemical colorimetric indicator. For example, the quantitative colorimetric measurement system can include a port configured to receive a water source, moisture source, or humidifier component. The introduction of water or moisture through the port can humidify the chemical colorimetric indicator and/or the gas passing through the system upstream of the chemical colorimetric indicator. In another example the introduction of the moisture or water can be used to wet a filter within the colorimetric system that can provide humidity to the gas passing through the system to humidify the chemical colorimetric indicator. The wet filter can also keep the chemical colorimetric indicator humidified during use of the system for a breathing therapy. The wet filter or other material can function as a humidity moisture exchanger (HME) to provide humidity to the air/gas passing through the wet filter.

In some embodiments humidifying the chemical colorimetric indicator includes contacting the first gas with a humidification source. For example, the first gas can be passed through a water or moisture source to mix the first gas with the water or moisture source to humidify the first gas and chemical colorimetric indicator. The water or moisture source can be provided with a removable cartridge containing the carbon dioxide calibration gas and the moisture source.

In some embodiments the humidification source can be provided with the reference gas. For example water or moisture can be provided within the container including the reference gas or in fluid communication with the gas conduit connecting the reference gas and chemical colorimetric indicator.

In some embodiments a hydrophilic super absorbent polymer (SAP) can be used to provide a humidity source.

The SAP can be in the gas container or in the gas pathway with the cartridge containing the chemical colorimetric indicator. In some embodiments the humidification source can be provided with the cartridge having the chemical colorimetric indicator.

In some embodiments the chemical colorimetric indicator can be optionally humidified after measuring the first color of the chemical colorimetric indicator and prior to exposing the chemical colorimetric indicator to the second gas. Humidifying the chemical colorimetric indicator prior to exposing the indicator to the second gas can be done by contacting the chemical colorimetric indicator with air exhaled by a user of the quantitative colorimetric measurement system or any of the humidification methods disclosed herein.

Exposing the chemical colorimetric indicator to a second gas can include exposing the indicator to a sealed container filled with a reference sample having a known carbon dioxide concentration. In some embodiments the known carbon dioxide concentration is from about 4% to about 7% carbon dioxide. In some embodiments the sealed container filled with the reference sample having the known carbon dioxide concentration has a known humidity content.

In some embodiments exposing the indicator to the first gas includes exposing the colorimetric indicator to a sealed container filled with a reference sample having a known carbon dioxide concentration. The sealed container filled with the reference sample having the known carbon dioxide concentration can have a known humidity content. In some embodiments the first gas has a concentration of CO2 of about 0% to about 2%. In some embodiments exposing the indicator to the first gas includes exposing the colorimetric indicator to ambient air. Ambient air can be used to provide a reference source having a near zero concentration of carbon dioxide to calibrate the lower end of the span calibration for the chemical colorimetric indicator. In some embodiments the span calibration can be applied to a measurement of a color of the chemical colorimetric indicator exposed to a breath sample.

The calibration methods can include engaging a cartridge with the quantitative colorimetric measurement system with the cartridge containing the reference gas and the chemical colorimetric indicator. The methods can further include the patient using the quantitative colorimetric measurement system for a breathing therapy for up to seven days. In some cases the methods include replacing the chemical colorimetric indicator with a second chemical colorimetric indicator after about 5-7 days.

In some embodiments the chemical colorimetric indicator can be used for greater than 5-7 days. For example, the chemical colorimetric indicator can be recalibrated about every 5-7 days with the colorimetric indicator used for a total time period of a month or longer. In some embodiments the chemical colorimetric indicator can be used for 28 days or longer. For each recalibration the calibration steps described herein can be used to re-calibrate the colorimetric indicator. The span calibration data developed by exposing the colorimetric indicator to a reference gas and ambient air can be used to recalibrate the chemical colorimetric indicator after a period of use following an earlier calibration. After recalibration the user can continue to use the quantitative colorimetric system to provide a breathing therapy. In some cases the recalibration of the chemical colorimetric indicator could skip the humidification step because the film would already have a threshold level of humidity from the prior use of the film for the breathing therapy.

The humidification and calibration methods described herein can be applied to each fresh or new chemical colorimetric indicator that is used in the system. The colorimetric indicator can become contaminated or lose the responsiveness needed for the applications disclosed herein. A new colorimetric indicator can be engaged with the colorimetric system and calibrated. For example the calibration methods can include humidifying the second chemical colorimetric indicator, exposing the second chemical colorimetric indicator to a first gas, measuring a first color of the second chemical colorimetric indicator based on the exposure to the first gas, exposing the second chemical colorimetric indicator to a second gas, measuring a second color of the second chemical colorimetric indicator based on the exposure to the second gas having a different $CO_2$ concentration than the first gas, and deriving a span calibration based on the difference between the first color of the second chemical colorimetric indicator and the second color of the second chemical colorimetric indicator. The calibration steps described herein can also determine when the chemical colorimetric indicator is performing poorly and needs to be replaced.

The methods disclosed herein can also include verifying the humidity of the chemical colorimetric indicator after humidifying the chemical colorimetric measurement system. Verifying the humidity of the chemical colorimetric indicator can also include measuring a color of a humidity sensor that is part of the colorimetric measurement system. In some embodiments verifying the humidity can include determining if the color of the humidity sensor corresponds to a humidity above a threshold humidity level. After the humidity of the chemical colorimetric indicator has been verified the span calibration or other calibration methods can be performed by exposing the chemical colorimetric indicator to the first and second gases.

In some embodiments a temperature adjustment or correction can also be applied to the interrogation of the color of the chemical colorimetric indicator. The methods described herein can include measuring a temperature of the chemical colorimetric indicator when measuring the color of the chemical colorimetric indicator. The method can further include applying a temperature adjustment or correction to the measured color based on the temperature of the chemical colorimetric indicator. For example the temperature can be used to calculate the carbon dioxide content of the sample gas in combination with the measured signal corresponding to the color of the chemical colorimetric indicator.

Initial characterization data determined using the various methods disclosed herein can be recorded for the tested chemical colorimetric indicator, in particular when the characterization is done prior to packaging the chemical colorimetric indicator. The methods can further include packaging the chemical colorimetric indicator. The initial characterization data for the chemical colorimetric indicator can be included with the packaged chemical colorimetric indicator. The characterization data can be encoded and included on or within the packaging for the chemical colorimetric indicator. The encoded information can be scanned or read by the quantitative measurement system or a hand held computing device in electronic communication with the quantitative measurement system, such as during the calibration procedure described above. Non limiting examples of encoding of the characterization data include: bar code, QR code, other machine readable, RFID, or cloud data transfer.

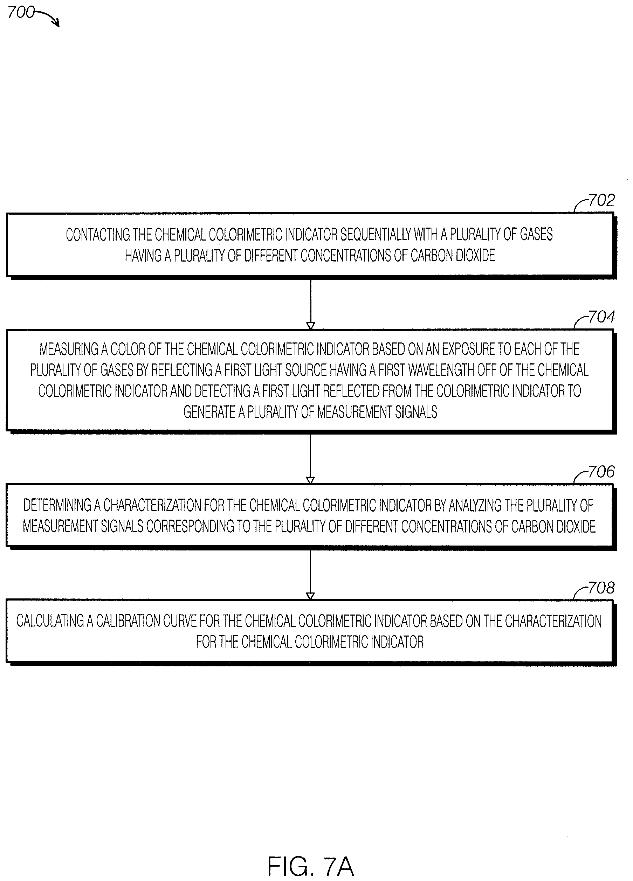

The initial characterization of the chemical colorimetric indicator can be used to determine a curve for the indicator's response to carbon dioxide. FIG. 7A is a flowchart illustrating a method 700 for characterizing a chemical colorimetric indicator in accordance with some embodiments. The method 700 includes contacting the chemical colorimetric indicator sequentially with a plurality of gases having a plurality of different concentrations of carbon dioxide 702 followed by measuring a color of the chemical colorimetric indicator based on an exposure to each of the plurality of gases by reflecting a first light source having a first wavelength off of the chemical colorimetric indicator and detecting a first light reflected from the colorimetric indicator to generate a plurality of measurement signals 704. Next a characterization for the chemical colorimetric indicator is determined by analyzing the plurality of measurement signals corresponding to the plurality of different concentrations of carbon dioxide 706. Then a calibration curve for the chemical colorimetric indicator can be calculated based on the characterization for the chemical colorimetric indicator.

The initial characterization of the indicator can include passing known carbon dioxide gas concentrations into contact with the indicator and measuring the response of the indicator. This is done for carbon dioxide concentrations from about 0% to about 8% CO2. The temperature dependence of the response of the indicator can also be observed. For example, the film can be subjected to multiple concentrations of carbon dioxide and a temperature controller can control the temperature of the indicator such that the indicator can be observed at multiple temperatures for each of the carbon dioxide concentrations. The indicator's response to the varying temperatures and carbon dioxide concentrations can be determined and considered the initial characterization of the film. The combination of the response data points to CO2 concentration and at various temperatures can then be turned into polynomial equations to describe the behavior of the indicator, such as converting a detected photodetector voltage to a corresponding carbon dioxide concentration. The characterization can include calculating and determining coefficients for the polynomial equations. Once film is characterized the coefficients can be used by a quantitative colorimetric measurement system as part of the calibration methods disclosed herein for the user to follow prior to using the device for a breathing therapy.

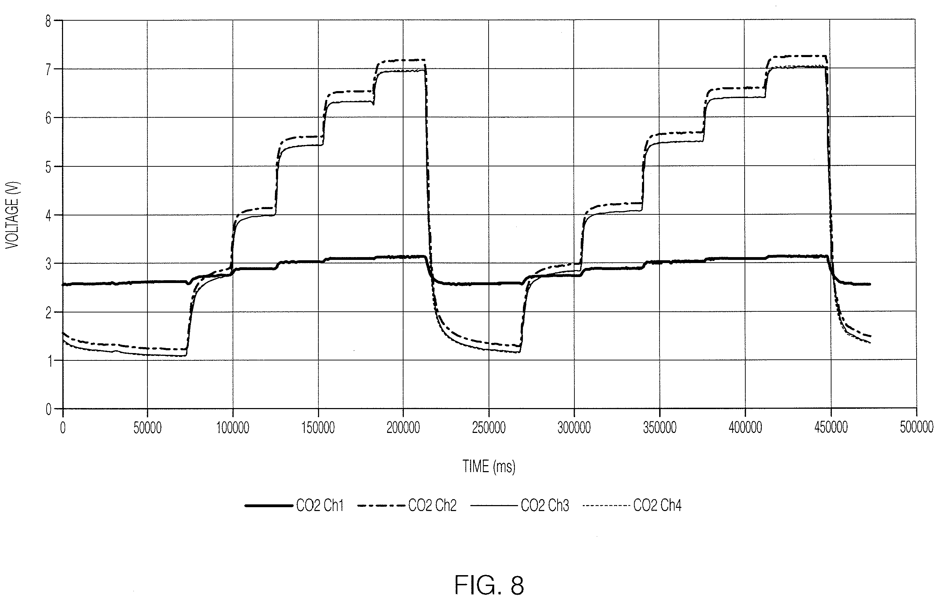

In some embodiments characterization of the indicator can include sequentially exposing the indicator to different concentrations of carbon dioxide across a range of temperatures within the operating range of the device. In one example the concentrations of carbon dioxide can be about 0, 1, 2, 4, 6, and 8% CO2 with temperatures across operating range (15-30°C). In some embodiments the plurality of temperatures are between about 15°C and 31°C. The corresponding voltage signal detected by the photodetector can be recorded for each of the different concentrations of carbon dioxide at each temperature and for each different wavelength or spectra reflected off of the chemical colorimetric indicator. FIG. 8 illustrates the measured voltage for four different LED colors while sequentially exposing the chemical colorimetric indicator to room air, 0%, 1%, 2%, 4%, 6%, and 8% carbon dioxide. FIG. 8 illustrates the different relationships between the voltage detected by a photodetector from different wavelengths of light reflecting off of the colorimetric indicator. In some embodiments each of the different wavelengths of light can be used to characterize the colorimetric indicator and derive coefficients used to model the indicator's response to carbon dioxide.

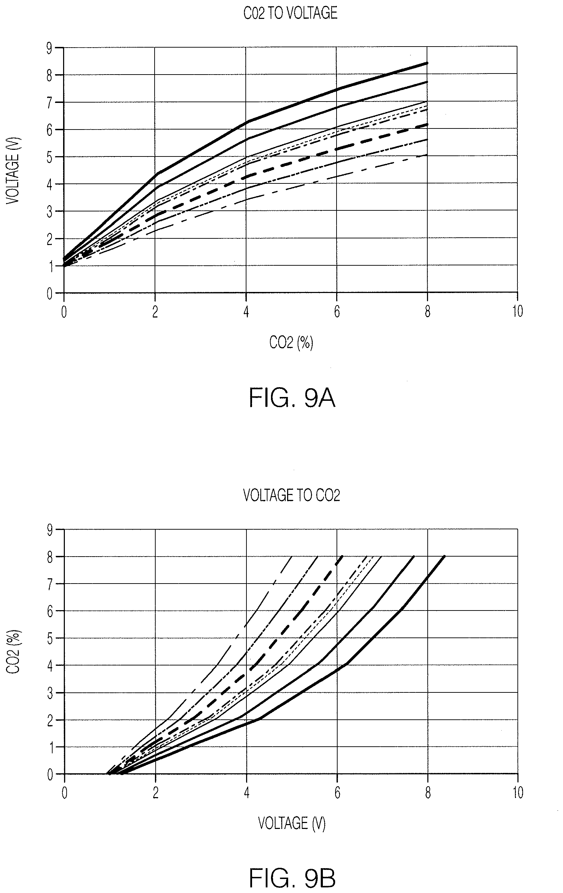

FIG. 9A illustrates a graph of voltage versus CO2 at various temperatures. FIG. 9B illustrates a graph of CO2 versus voltage at various temperatures. The measured voltages (V) at each CO2 concentration and temperature (T) are recorded. The measured voltages can be used to calculate fit coefficients for a polynomial equation that can be used to convert the measured voltage and temperature to a corresponding carbon dioxide concentration.

In some embodiments the function can be represented as: $CO2(V) = [A' * V^3] + [B' * V^2] + [C' * V] + D'$. Each of A', B', C' and D' can be a function of temperature. In some embodiments the function can be represented as: $CO2(V,T) = [A'(T) * V^3] + [B'(T) * V^2] + [C'(T) * V] + D'(T)$ with $A'(T) = a_1 * T^2 + b_1 * T + c_1$; $B'(T) = a_2 * T^2 + b_2 * T + c_2$ ; $C'(T) = a_3 * T^2 + b_3 * T + C_3$ ; and $D'(T) = a_4 * T^2 + b_4 * T + c_4$.

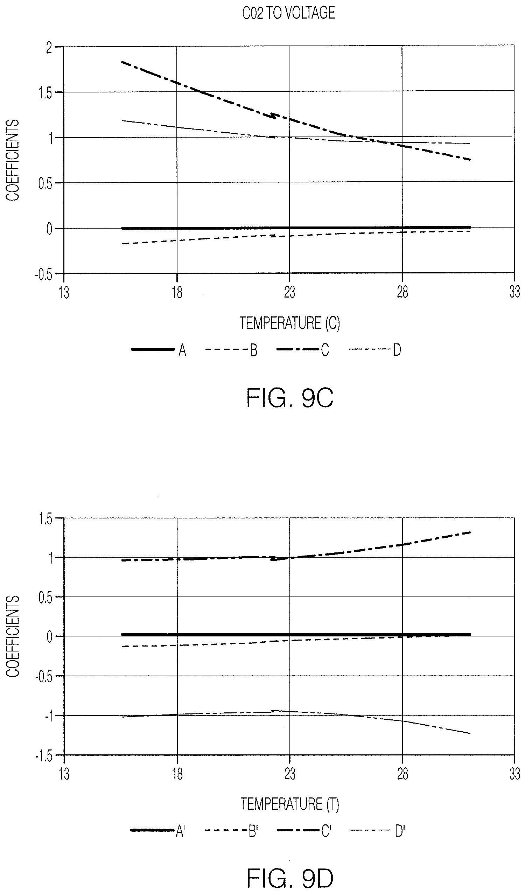

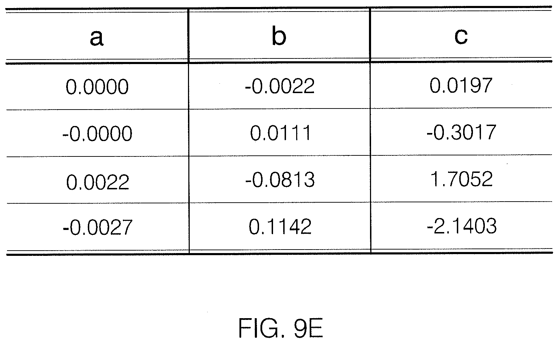

FIG. 9C illustrates a graph of coefficients versus temperature for the CO2 to voltage data of FIG. 9A. FIG. 9D illustrates a graph of coefficients versus temperature for the voltage to CO2 of FIG. 9B. FIG. 9E illustrate corresponding fit coefficients in accordance with some embodiments. The initial characterization of the film can be used to calculate a1, a2, a3, a4, b1, b2, b3, b4, c1, c2, c3, and c4 as illustrated in the examples shown in FIGS. 9A-9E.

In some embodiments the humidity of the chemical colorimetric indicator can be considered when interrogating the chemical colorimetric indicator. The humidity of the film can be determined using a humidity sensor, humidity probe, or other humidity detection device. The humidity sensor can be included within the device, such as on one or more of the printed circuit boards (PCB) on the device and/or removable cartridges. The humidity of the chemical colorimetric indicator can be applied to the voltage corresponding to the color of the chemical colorimetric indicator when the voltage is measured. In other embodiments the humidity can be applied as a humidity correction after obtaining the voltage measurement.

In some embodiments the humidity can be accounted for by using the following equation:

$$V_{RH}(\text{RH}) = V_{Meas} * \left( \frac{1}{p*RH^2+q*RH+r} \right)$$

In some embodiments the measured voltage (i.e., color response) is multiplied by the relative humidity (RH) factor in the equation above, where p, q, and r are coefficients of the parabolic function and RH is the measured relative humidity in the system. The compensation can be used to normalize the voltage measured to correspond to an effective relative humidity of 60%.

The humidity application can be applied to all of the methods for interrogating the chemical colorimetric indicator described herein. For example, the humidity of the film can be applied to voltage measurements during the characterization of the film, calibration of the film, and during use of the system by the user.

The film characterization and coefficient calculation can be performed for each batch of film. The coefficients can be encoded with the packaging with a barcode, RFID, or other machine readable format. In some embodiments the coefficients can be stored in the cloud and downloaded by the system when the chemical colorimetric material is used.

The characterization and calibration methods can also include measuring a temperature of the chemical colorimetric indicator when measuring the color of the chemical colorimetric indicator based on the exposure to the first gas. The methods can also include applying a temperature correction to the first measurement signal based on the temperature of the chemical colorimetric indicator when measuring the color of the chemical colorimetric indicator.

Any of the characterization data determined in the laboratory or factory can be encoded with the colorimetric material as described herein. The properties and characteristics of the film, such as the response to carbon dioxide, can change over time when the film is stored. In some embodiments the colorimetric material can be re-calibrated prior to use by the patient.

The methods can include the user removing the colorimetric chemical indicator from a package containing the colorimetric indicator. The package containing the colorimetric indicator can include the factory characterization data for the colorimetric chemical indicator. The factory characterization data can be encoded such that a hand held computer or the quantitative colorimetric measurement system can scan or read the characterization data. The factory characterization data can refer to the characterization determined for the specific chemical colorimetric indicator determined at the factory or at the lab prior to packaging the indicator.

The user can engage the colorimetric chemical indicator with the quantitative colorimetric measurement system. After calibrating the chemical colorimetric material in the quantitative colorimetric measurement system the system is ready for use by the user. For example, the user can perform any of the methods and breathing therapies disclosed herein. In some embodiments the user can then humidify the chemical colorimetric indicator prior to exposing the chemical colorimetric indicator to the first gas.

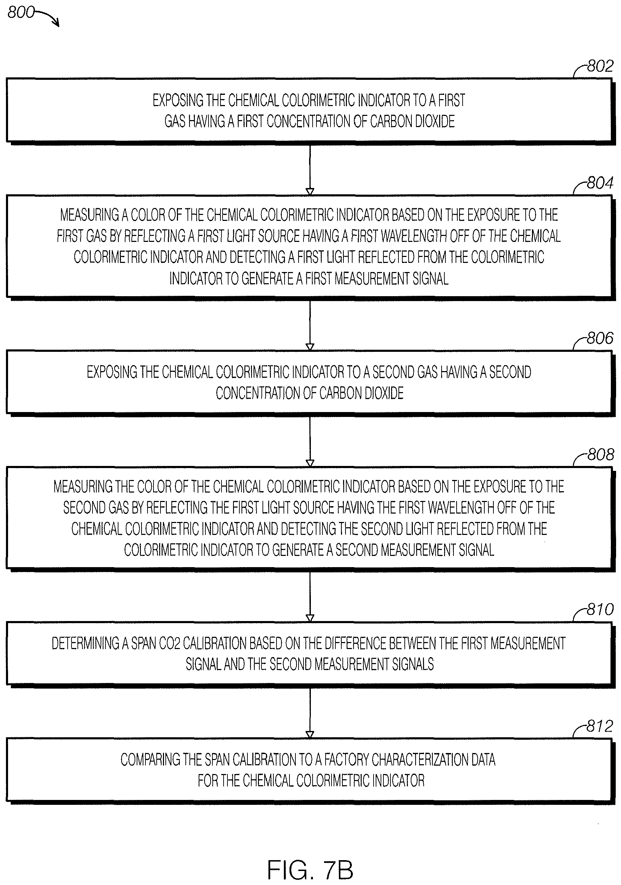

The factory characterization data for the chemical colorimetric indicator can be compared to any of the calibration data developed using the methods disclosed herein. The calibration data and fit coefficients can be compared to the factory calibration and characterization data followed by adjusting the calibration data or curve for the film based on the calibration data at the time of use. FIG. 7B is a flowchart illustrating a method 800 for calibrating a chemical colorimetric indicator in accordance with some embodiments. The method can include exposing the chemical colorimetric indicator to a first gas having a first concentration of carbon dioxide 802, measuring a color of the chemical colorimetric indicator based on the exposure to the first gas by reflecting a first light source having a first wavelength off of the chemical colorimetric indicator and detecting a first light reflected from the colorimetric indicator to generate a first measurement signal 804, exposing the chemical colorimetric indicator to a second gas having a second concentration of carbon dioxide 806, measuring the color of the chemical colorimetric indicator based on the exposure to the second gas by reflecting the first light source having the first wavelength off of the chemical colorimetric indicator and detecting the second light reflected from the colorimetric indicator to generate a second measurement signal 808, determining a span CO2 calibration based on the difference between the first measurement signal and the second measurement signals 810, and comparing the span calibration to a factory characterization data for the chemical colorimetric indicator 812.

The calibration of the quantitative colorimetric measurement system can be adjusted based on the span calibration and factory characterization data. In some embodiments the characteristics and properties of the light sources can be adjusted such that the voltage response of the chemical colorimetric indicator to the reference gas and ambient air substantially matches the characterization data. In some embodiments other adjustments can be made to the system to match the voltage response of the chemical colorimetric indicator to the reference gas and ambient air to the characterization data. For example, one or more of the system signal processing, photodetectors, or other aspects of the system can be modified. Modifying the system properties to match the voltage response of the chemical colorimetric indicator to the characterization data can allow for the original characterization fit coefficients to be used by the system.

In some embodiments the system can perform an optimization of the LED to achieve similar voltage readings to the characterization for the reference gas concentration and ambient air. For example the derived coefficients and calibration curves can be used to calculate the carbon dioxide concentration from the measured voltage readings for the reference gas and the ambient air. The system hardware and software can then modify the LED drive to match the detector voltage levels based on the characterization data for the ambient air and reference gas at the recorded temperature. In some embodiments optimizing the LED drive can include matching the voltage difference between the ambient air and reference gas at the recorded temperature. The adjustments in the drive, offsets, and gains for the LED can be made by the system such that the original characterization fit coefficients can be used to calculate the CO2 concentration based on the measured voltages and temperatures. In some embodiments modifying the LED drive can include adjusting the electrical current supplied to the light source/LED.

In some embodiments the factory characterization data can include a span calibration for carbon dioxide exposure to the chemical colorimetric indicator. The span calibration can be as described herein, including humidification of the colorimetric indicator and exposure to gases with varying carbon dioxide concentrations. The methods can include comparing the span CO2 calibration to the calibration data and/or factory characterization data. The calibration curve for the colorimetric indicator can be adjusted based on the comparison of the factory characterization data with calibration data prior to using the colorimetric indicator.

In some embodiments temperature corrections can also be applied during the factory characterization and calibration performed just prior to using the colorimetric indicator in the quantitative measurement system.

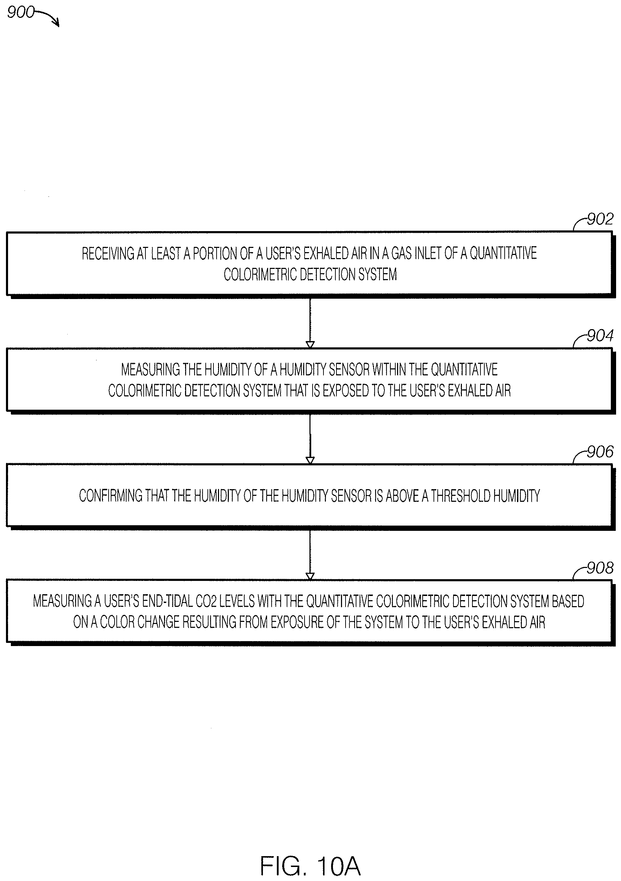

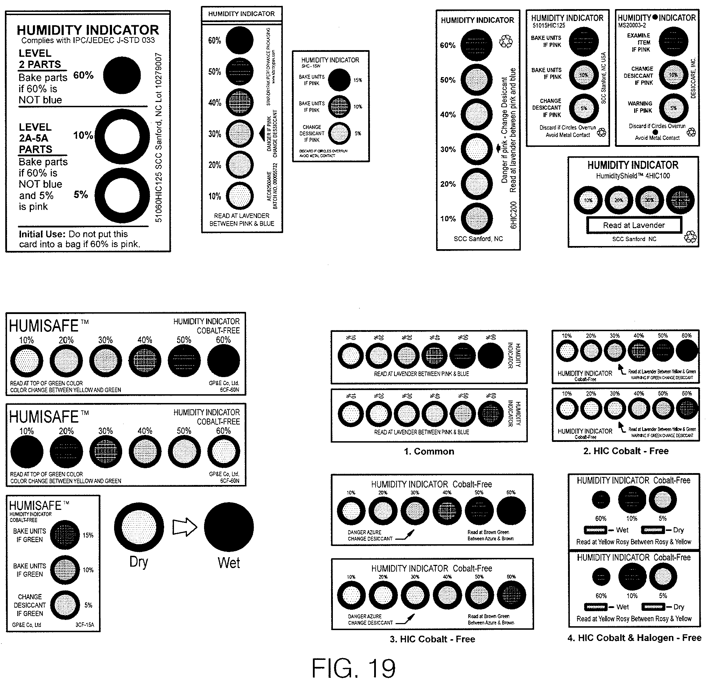

FIG. 10A is a flowchart illustrating a method 900 for providing a breathing therapy in accordance with some embodiments. The breathing therapy methods 900 can include receiving at least a portion of a user's exhaled air in a gas inlet of a quantitative colorimetric measurement system 902, measuring the humidity of a humidity sensor within the quantitative colorimetric measurement system that is exposed to the user's exhaled air 904, confirming that the humidity of the humidity sensor is above a threshold humidity 906, and measuring a user's end-tidal CO2 levels with the quantitative colorimetric measurement system based on a color change resulting from exposure of the system to the user's exhaled air 908. A humidity sensor can be used to confirm that the colorimetric indicator is above a threshold humidity and that the colorimetric indicator is ready to be used for a breathing therapy. In some embodiments a humidity sensor can be used that changes color in response to various humidity levels. A variety of examples of humidity sensors that change color in response to humidity are illustrated in FIG. 19. The photodetector can interrogate the humidity sensor to determine the color of the humidity sensor. The interrogated color of the humidity sensor can be compared to the color of the humidity sensor at the threshold humidity level.

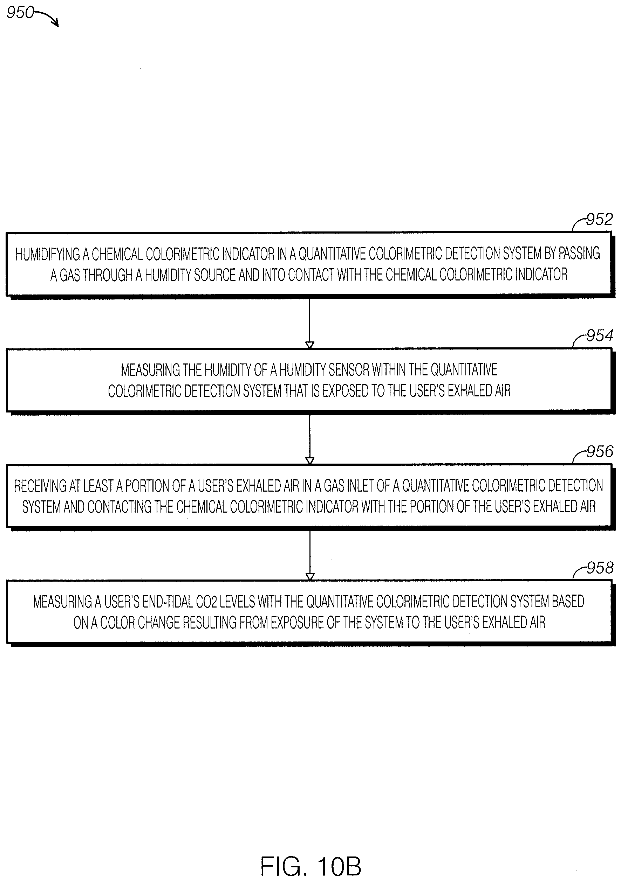

FIG. 10B is a flowchart illustrating a method 950 for providing a breathing therapy in accordance with some embodiments. The breathing therapy methods 950 can include humidifying a chemical colorimetric indicator in a quantitative colorimetric detection system by passing a gas through a humidity source and into contact with the chemical colorimetric indicator 952, measuring the humidity of a humidity sensor within the quantitative colorimetric detection system that is exposed to the user's exhaled air 954, receiving at least a portion of a user's exhaled air in a gas inlet of a quantitative colorimetric detection system and contacting the chemical colorimetric indicator with the portion of the user's exhaled air 956, and measuring a user's end-tidal $CO_2$ levels with the quantitative colorimetric detection system based on a color change resulting from exposure of the system to the user's exhaled air 958. A humidity sensor can be used to measure the humidity of the colorimetric indicator. The humidity of the colorimetric indicator can be used to determine the carbon dioxide in the user's exhaled breath samples as described herein. The humidity sensor can confirm that the colorimetric indicator is above a threshold humidity and that the colorimetric indicator is ready to be used for a breathing therapy. Examples of humidity sensors include electronic and colorimetric humidity sensors.

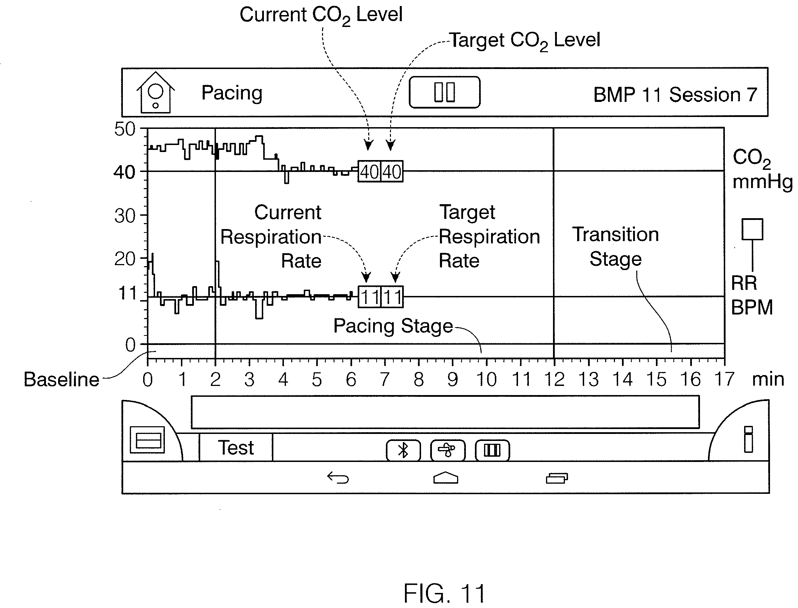

FIG. 11 illustrates a graphical representation of end-tidal CO2 and breathing rate in accordance with some embodiments. The program may display a graph showing the end-tidal CO2 levels and breathing rate with goal lines for target values. FIG. 11 shows goal line (dashed) CO2 pressure at 40 mmHg and goal line (dashed) 13bpm for breathing rate. In some embodiments, the system may provide the patient with advice or tips during the session on how to reach goals such as raising end-tidal CO2. As shown in FIG. 11, current CO2 levels are indicated in a box that shows the CO2 level of the patient's last breath. The line leading up to the box shows a record of the patient's CO2 level during the current breathing session. The box next to the Current CO2 level shows the Target CO2 level, which is 37-40 mmHg ("millimeters of Mercury") in the example. The current Respiration Rate (RR) can be displayed in another box. The line leading up to the box showing the RR can show a record of the patient's RR during the current breathing session. The box next to the Current Respiration Rate shows the Target Respiration Rate.

The breathing therapy methods can include outputting a set of visual and/or audio cues from the quantitative colorimetric system with instructions for the user to adjust their breathing pattern to coincide with the cues to thereby modify the user's exhaled CO2 levels. In some embodiments the breathing pattern includes the exhaled CO2 level and respiration rate. In some embodiments displaying the user's measured CO2 levels to provide visual feedback during treatment. In some embodiments displaying the user's breathing rate to provide visual feedback during treatment. In some embodiments the therapy directs the user's end-tidal CO2 levels to a level between about 37 mmHg and 43 mmHg.

The breathing therapy methods can include treating post-traumatic stress disorder (PTSD), panic disorder, anxiety, asthma, hypertension, obsessive-compulsive disorder, social phobia, depression, apnea, migraines, or epilepsy by training the user to modify their exhaled CO2 levels. In some embodiments the temperature of the chemical colorimetric indicator can be measured during the breathing therapy. A temperature correction can be applied to the measured voltage.

In addition to the above, various aspects of the inventions are directed to a breathing therapy system for non-invasively and non-pharmaceutically treating various conditions include panic disorder, anxiety, general anxiety disorder, obsessive-compulsive disorder, social phobia, depression, apnea, migraines, epilepsy, asthma, post-traumatic stress disorder, and hypertension. Some embodiments described herein are directed toward breathing therapy to treat a disorder or disease. For example, quantitative colorimetric carbon dioxide measurement system described can be used to measure and modify a user's CO2 levels to provide treatment for any number of disorders or illnesses.

A variety of different configurations for cartridges including colorimetric chemical indicators are also disclosed herein. The cartridge can include a gas measurement chamber and the chemical colorimetric indicator. Some embodiments include a cartridge comprising a colorimetric chemical indicator with the cartridge configured to removably engage with a quantitative colorimetric measurement system and a sealed container comprising a reference gas comprising a known concentration of carbon dioxide. In some embodiments the known carbon dioxide concentration can be from about 4% to about 7% carbon dioxide.

In some embodiments the sealed container is configured for a single use. In some embodiments the sealed container can be configured for multiple uses. In some embodiments the sealed container is configured to be resealable. In some embodiments the sealed container is integral with the cartridge. In some embodiments the sealed container is separate from the cartridge. In some embodiments the sealed container contains a known humidity content.

In some embodiments a reusable sealed container with the reference gas can be part of a reference gas station. The reference gas station can be used to provide the reference gas to the quantitative colorimetric measurement system by the user. The reference gas station can dock with the system to provide the reference gas. The gas station can be used multiple times to calibrate the quantitative colorimetric measurement system. In some cases the reference gas station can also include a humidifier. The humidifier can be used to humidify the colorimetric indicator.

The reusable sealed container can have a volume sufficient to hold enough gas to calibrate the chemical colorimetric material one or more times. In some embodiments the reusable sealed container has a volume of greater than about 50 ml. In some embodiments the reusable sealed container has a volume that is less than about 100 ml.

In some embodiments the cartridge can include a second sealed container having a second known concentration of carbon dioxide. The second known concentration of carbon dioxide can be less than about 2% carbon dioxide. In some cases the second sealed container contains a known humidity content.

In some embodiments the cartridge can include a humidification module configured to humidify an incoming gas prior to the gas contacting the colorimetric chemical indicator. In some embodiments the humidification module includes a humidity reservoir. In some embodiments the humidification module includes a wet filter, hydrophilic absorbent material, super absorbent polymer (SAP), or humidity-moisture exchanger (HME). In some embodiments the humidification module includes a water reservoir.

In some embodiments the cartridge includes a humidity sensor. FIG. 19 illustrates various colorimetric humidity sensors in accordance with some embodiments. In some embodiments electronic humidity sensors can be used. The humidity sensor can be used by the quantitative colorimetric measurement system to determine if the colorimetric indicator exceeds a threshold humidity level and/or to determine the humidity level of the colorimetric as part of interrogating the colorimetric indicator to determine the carbon dioxide content.

The cartridges can be used with the quantitative colorimetric measurement systems disclosed herein. The quantitative colorimetric measurement system can include an electro-optical sensor assembly including one or more light sources and a photodiode configured to detect light reflected off of the chemical colorimetric indicator by the light source. The electro-optical sensor assembly can be configured to generate an electrical signal based on the light detected by the photodiode. The system can include a processor in communication with the electro-optical sensor assembly. The processor can be configured to receive the electrical signals generated by the electro-optical sensor assembly. The processor can use the signals to compute the quantity of carbon dioxide exposed to the chemical colorimetric indicator. The processor can be integral with the quantitative colorimetric measurement system or part of a hand held computing device.

The chemical colorimetric indicator is adapted to change color in response to exposure to a quantity of carbon dioxide gas. In some embodiments the colorimetric indicator uses m-cresol purple. In some embodiments the chemical colorimetric indicator can be formulated or configured to change color in response to other chemicals. For example, the chemical colorimetric indicator can be configured to measure the amount of carbon dioxide in a solution with water or other solvent. In some cases the chemical colorimetric indicator can be configured to measure hydrocarbons (CxHy).

The electro-optical assembly can also be configured to detect light reflected off of a humidity sensor that is part of the removable cartridge or other part of the quantitative colorimetric measurement system. The humidity sensor can be adapted to change color in response to exposure to humidity. In some embodiments the humidity sensor is downstream of the colorimetric chemical indicator. In some embodiments the humidity sensor is upstream of the colorimetric chemical indicator.

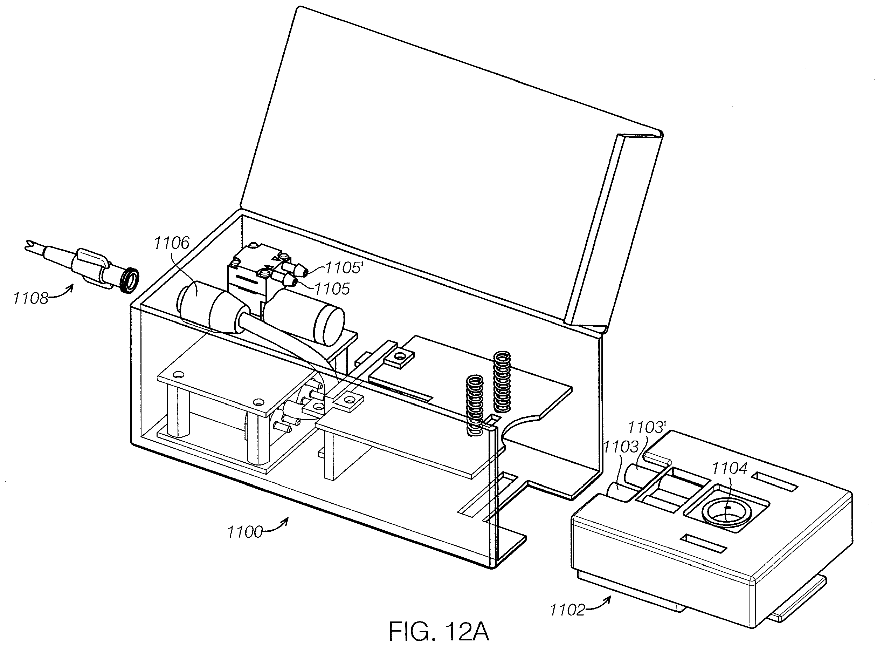

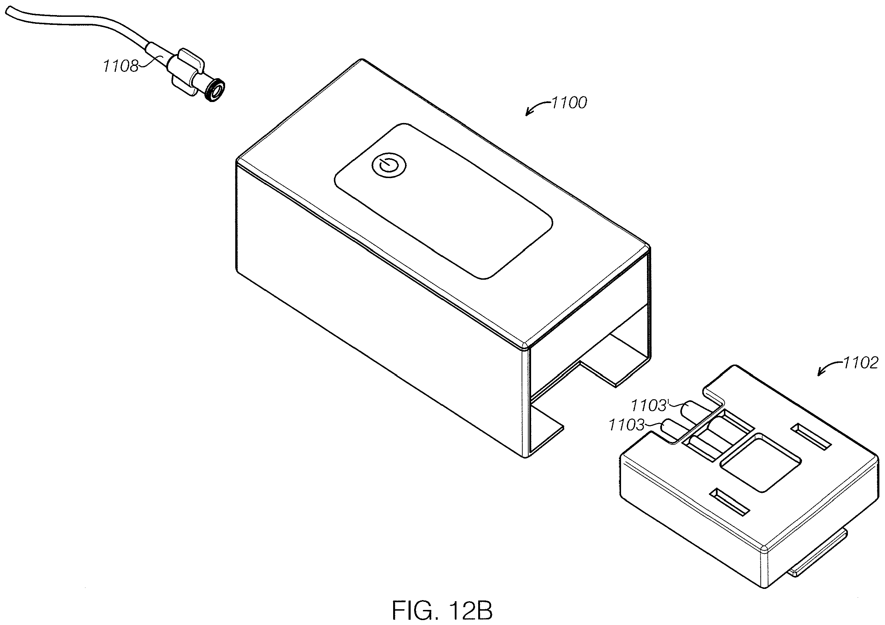

FIGS. 12A-12B illustrate a quantitative colorimetric measurement system 1100 including a removable cartridge 1102 with an inlet 1103, outlet 1103', and a chemical colorimetric indicator 1104 in accordance with some embodiments. The inlet 1103 and outlet 1103 are configured to engage with complementary surfaces 1105 and 1105' of the system 1100. The system 1100 includes a gas inlet 1106 configured to receive a sample tube 1108. The user's exhaled air is introduced to the system 1100 through the sample tube 1108 and gas inlet 1106 by a vacuum pump. The user's exhaled air is routed by the system 1100 through the inlet 1103 into contact with the chemical colorimetric indicator 1104. The color of the chemical colorimetric indicator 1104 can be interrogated by the LEDs or other light sources with the reflected light converted to a voltage measurement by a photodetector. The removable cartridge 1102 can be replaced when the chemical colorimetric indicator 1104 is chemically altered and less responsive.

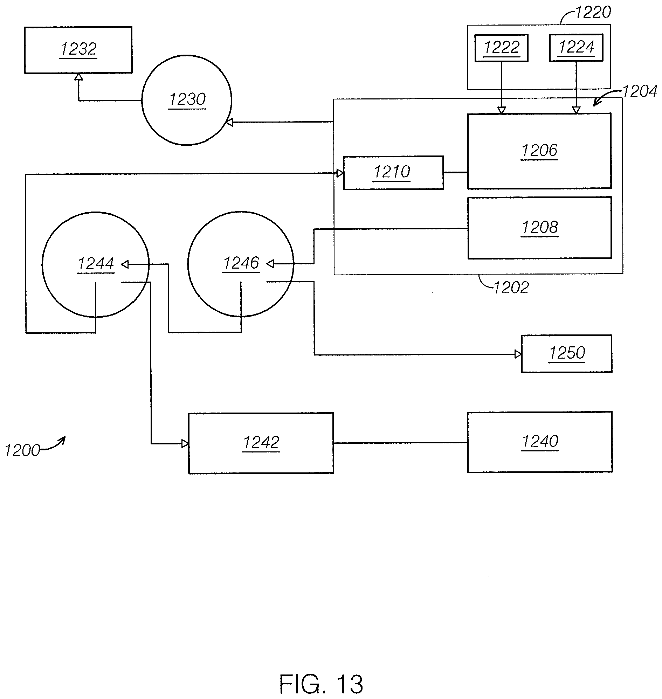

FIG. 13 illustrates a block diagram of quantitative colorimetric measurement system 1200 with a removable cartridge 1202 with a chemical colorimetric indicator 1204 in accordance with some embodiments. The removable cartridge 1202 or removable cell can include a gas measurement chamber 1206, a chemical colorimetric indicator 1204, a calibration gas 1208, and a filter or moisture trap 1210. The filter or moisture trap 1210 can be used to block solid and liquid contaminants from contacting the colorimetric indicator. The system 1200 can include an electro-optical assembly 1220 with a photodetector 1222 and LEDs 1224. The system can also include a pump 1230 to direct the sample to the outlet 1233. The exhaled breath sample travels through a patient cannula 1240 and sample inlet 1242. The calibration gas and sample gas can be routed through the internal plumbing of the system 1200 with a pair of three way solenoid valves 1244, 1246. The system 1200 can include an optional room air inlet 1250.

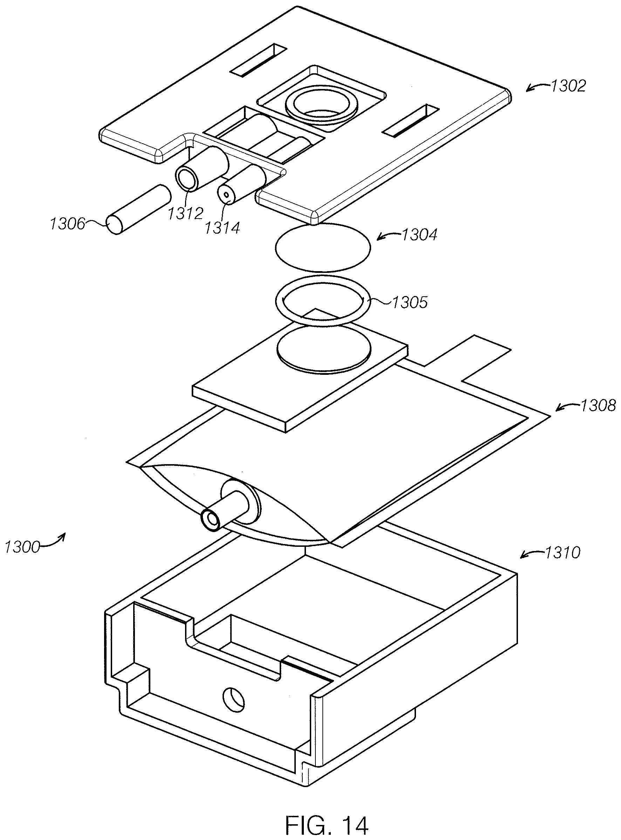

FIG. 14 illustrates is an isometric illustration of the components of a removable cartridge 1300 for use with a quantitative colorimetric measurement system in accordance with some embodiments. The cartridge 1300 includes a measurement chamber or gas cell 1302, chemical colorimetric indicator 1304, O-ring 1305, filter 1306, calibration gas pouch 1308, and housing 1310. The cartridge 1300 includes an inlet 1312 and outlet 1314.

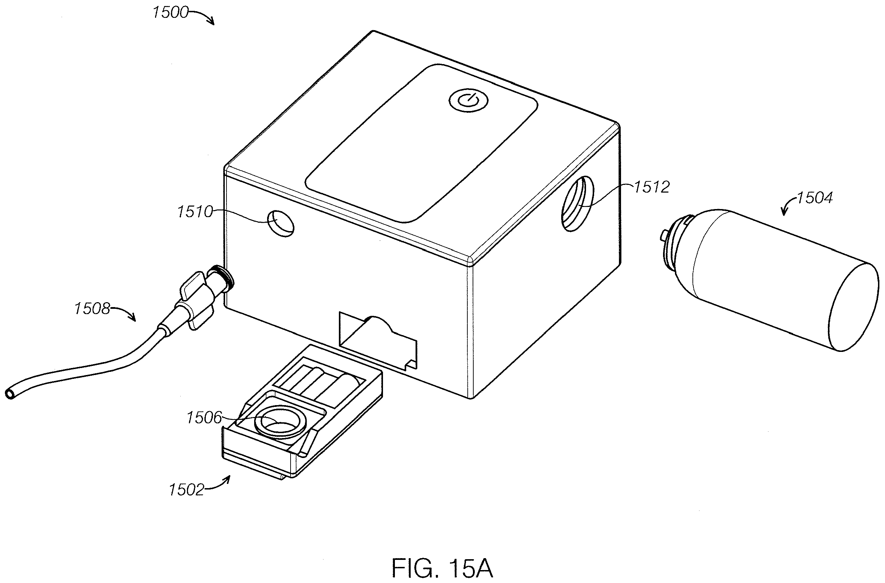

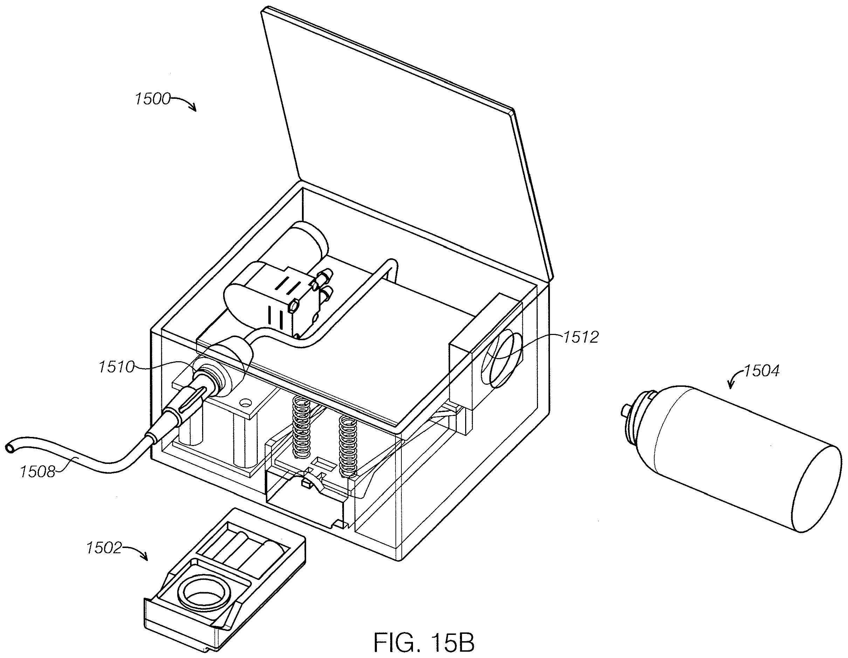

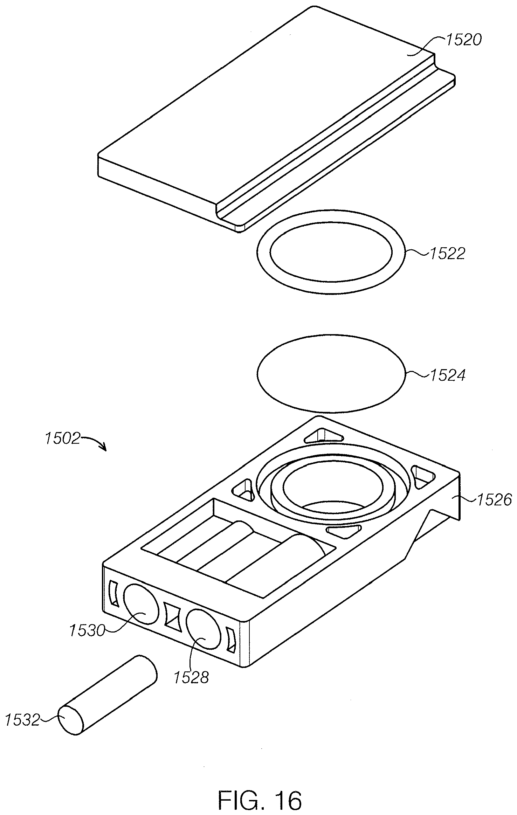

FIGS. 15A-15B illustrate a quantitative colorimetric measurement system 1500 including a removable cartridge 1502 and calibration gas container 1504 with a chemical colorimetric indicator 1506 in accordance with some embodiments. A sample tube or cannula 1508 is configured to engage with the gas inlet 1510 to introduce the air sample. The calibration gas container 1504 is configured to be received by an opening 1512. FIG. 16 is an isometric illustration of the components of the removable cartridge 1502 for use with a quantitative colorimetric measurement system in accordance with some embodiments. The cartridge 1502 includes a cover 1520, O-ring 1522, chemical colorimetric indicator 1524, and gas cell 1526. The cartridge 1502 includes a gas inlet 1528 and gas outlet 1530. The cartridge 1502 also includes a filter 1532 on the gas inlet 1528 to catch particulate matter, liquid, and other contaminates.

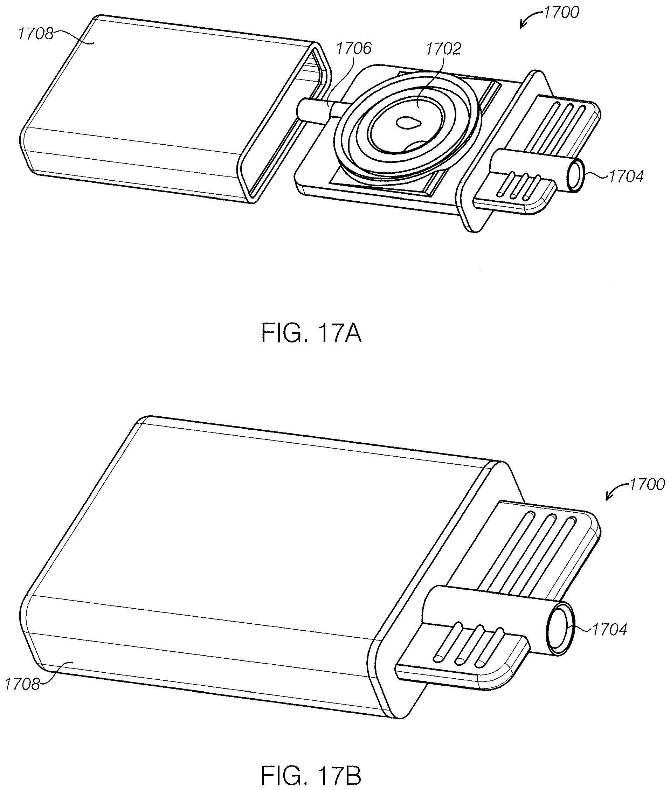

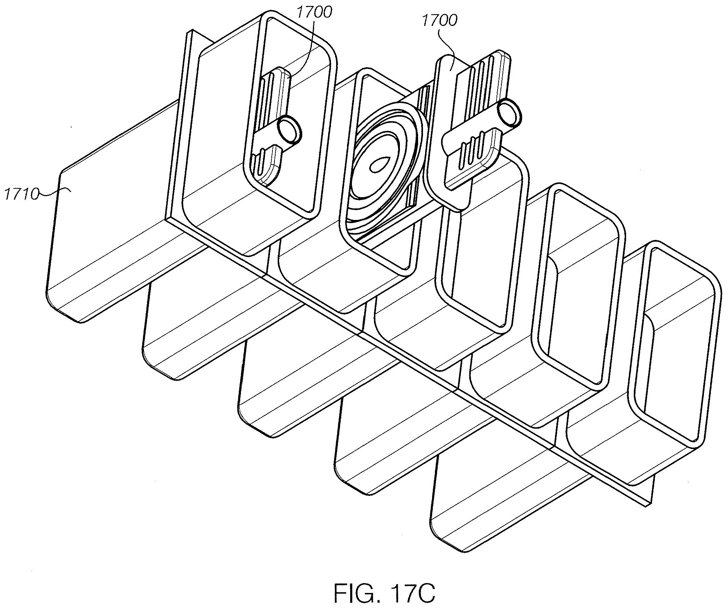

FIGS. 17A-17C illustrate removable cartridges 1700 including a chemical colorimetric indicator 1702 in accordance with some embodiments. The cartridge 1700 include the indicator 1702 with a gas inlet 1704 and gas outlet 1706 on opposite sides. The cartridge 1700 can be provided in a packaging container 1708. The packaging container can prevent moisture and other contaminants from interacting with the chemical colorimetric indicator prior to use. In some embodiments the packaging can be a material with a low vapor transmission, such as a metalized bag. The packaging can further include a desiccant to keep the chemical colorimetric indicator dry. FIG. 17C illustrates the removable cartridge 1700 in a multiple packaging container 1710.

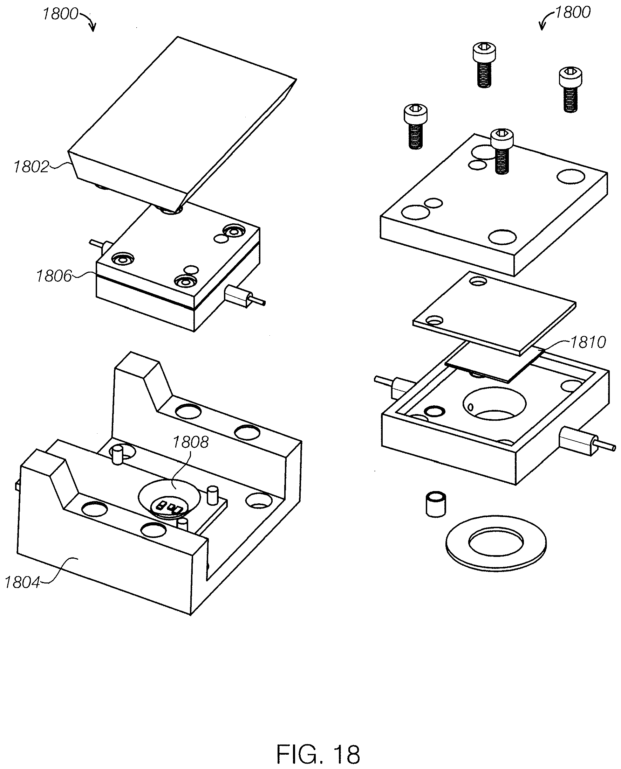

FIG. 18 illustrates aspects of quantitative colorimetric measurement systems 1800 in accordance with some embodiments. The system 1800 can include an isothermal chamber to provide tighter temperature coupling between the chemical colorimetric indicator 1810, LEDs, photodetector, and any temperature control unit. The isothermal chamber can be formed from a top lid 1802 and bottom housing 1804 to enclose the optical chamber 1806 and photodetector 1808. The system 1800 can use LEDs with four or more different wavelengths. The right side shows the exploded view of the optical chamber 1806 construction of the quantitative colorimetric measurement sensor. The top goes over a gasket material that compresses a chemical colorimetric indicator in the optical chamber. A 'donut' gasket is illustrated that can seal the optical chamber and the PCB together so no gas leaks in or out. A small indexing sleeve is also illustrated to assist with positioning. In the left image the optical chamber fits over the PCB. The illustrated optical chamber and PCB can be indexed with guide pins. The optical chamber can be held down with a top that includes some strong magnets to hold it in place.

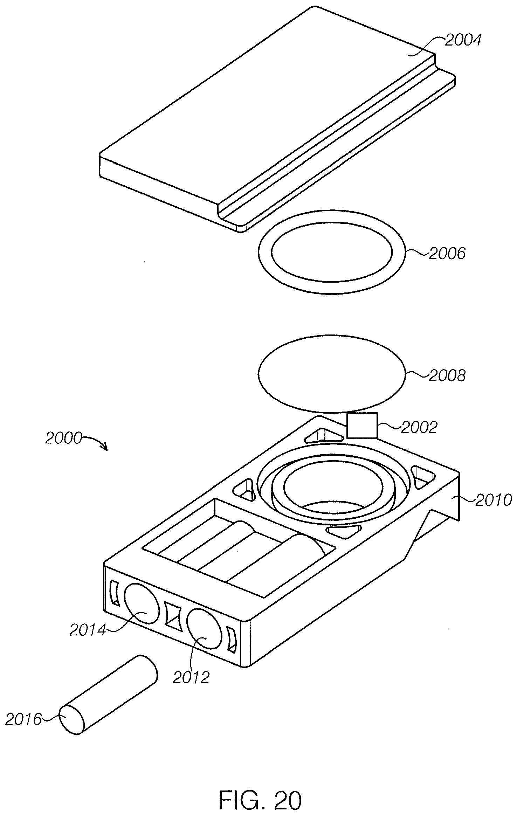

FIG. 20 is an isometric illustration of the components of a removable cartridge 2000 for use with a quantitative colorimetric measurement system including a humidity sensor 2002 in accordance with some embodiments. The cartridge includes a cover 2004, O-ring 2006, chemical colorimetric indicator 2008, humidity sensor 2002, and gas cell 2010. The cartridge 2000 also includes a gas inlet 2012 and gas outlet 2014. The cartridge also includes a filter 2016 on the gas inlet to catch particulate matter, liquid, and other contaminates.

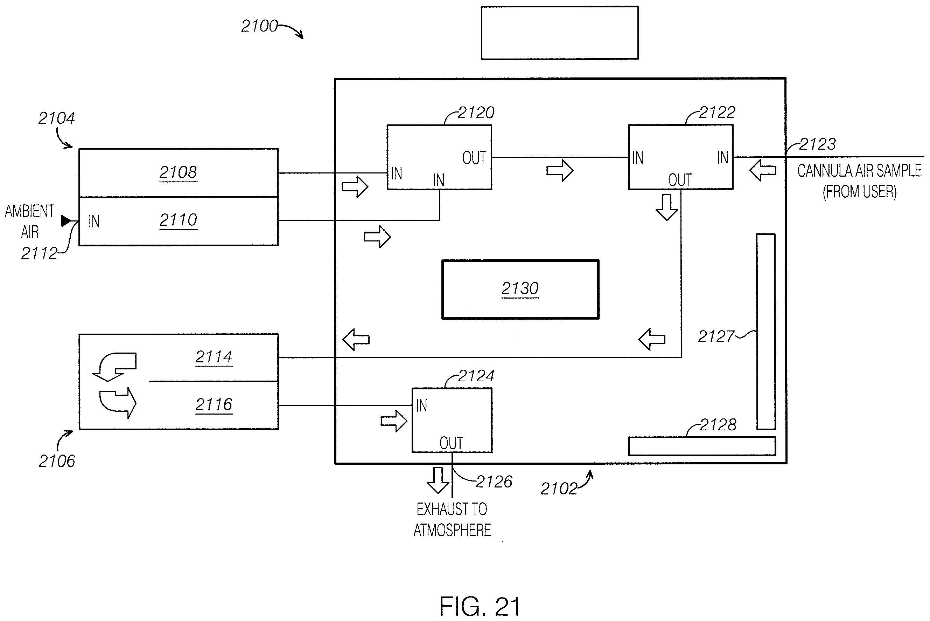

FIG. 21 is a schematic diagram of a quantitative colorimetric measurement system 2100 in accordance with some embodiments. The system 2100 includes a quantitative colorimetric measurement device 2102 configured to receive a calibration module 2104 and film assembly 2106. FIG. 21 illustrates a replaceable calibration module 2104 that includes a carbon dioxide canister 2108, a humidity source 2110, and an ambient air inlet 2112. The film assembly 2106 includes a humidity moisture exchanger (HME) 2114 and printed circuit board (PCB) with a chemical colorimetric indicator film 2116. Ambient air can be pulled in through the calibration module 2104 and the humidity source 2110 to humidify the ambient air. The humidified ambient air can be routed through the system 2102 and into contact with the chemical colorimetric indicator film 2116 contained in the second cartridge containing the film cell assembly 2106. For example, the three way solenoid valves 2120, 2122 can be used to route the desired gas flow into contact with the film. The illustrated miniature pump 2124 can be used to pull the desired gas through the device and out the exhaust into the atmosphere external to the system. The first three-way valve 2120 can allow either the humidified ambient air or the carbon dioxide reference gas to pass through the outlet of the first three-way valve 2120. The second three-way valve 2122 can allow either the breath sample from the user (from the inlet for the cannula 2123) or the gas passing through the first three-way valve 2120 to pass through the outlet of the second three-way valve 2122. The gas exiting the second three-way valve 2122 can be passed through a humidity moisture exchanger 2114 and into contact with the chemical colorimetric indicator film 2116 prior to passing through the pump 2124 and out the exhaust 2126. The illustrated colorimetric measurement device 2102 includes a power source, such as a battery 2127 and an optional display 2128. The colorimetric measurement device 2102 includes a PCB 2130 with a processor Bluetooth transmitter and controller. In some embodiments the system can wirelessly transmit data (e.g. with Bluetooth) to a computer display device, such as a laptop, tablet computer, smartphone, or desktop computer.

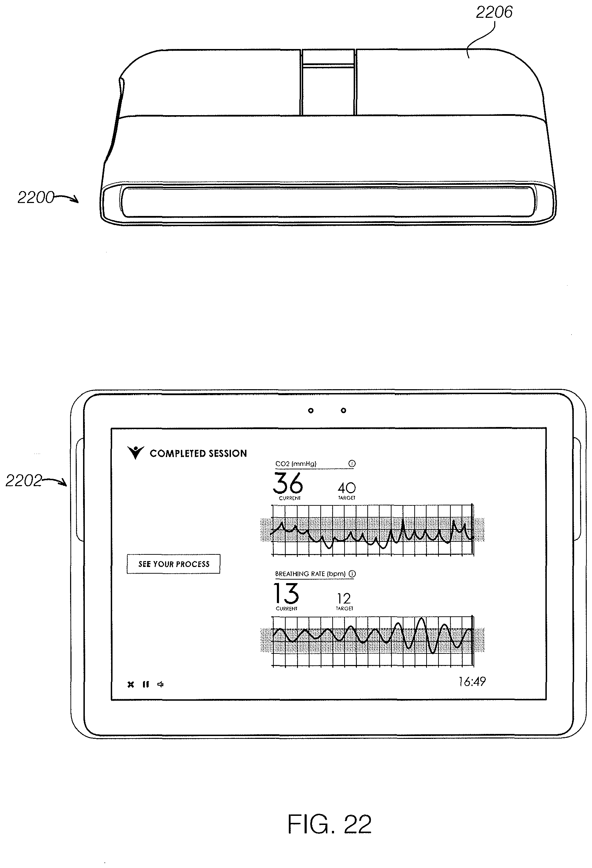

FIG. 22 illustrates a quantitative colorimetric measurement system 2200 with a computer display device 2202 in accordance with some embodiments. The system 2200 can include all of hardware illustrated in the schematic in FIG. 21. FIG. 22 shows the system 2200 wirelessly transmitting data to the computer display device 2202, with the computer display device 2202 showing real time user data on the display. The real time user data includes the $CO_2$ in the exhaled breath and the respiration rate.

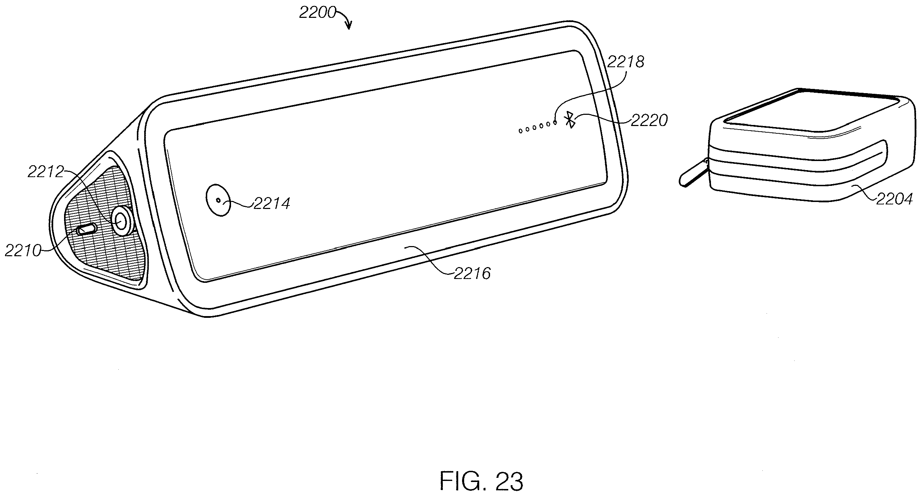

FIG. 23 illustrates a quantitative colorimetric measurement system 2200 including a cannula case 2204 in accordance with some embodiments. The illustrated system 2200 shows a USB port 2210 and a cannula connection 2212 on one side. The USB port 2210 can be used to make a wired data connection with the system 2200 to send and receive data. The cannula connection 2212 is configured to receive a cannula carrying the user's exhaled breath. The illustrated system includes a power button with a status indicator 2214, LED ring 2216, status indicator 2218, and Bluetooth indicator 2220 on another side. The status indicator 2218 can include a plurality of LEDs each adapted to display one or more different colors of light. The status indicator 2218 can provide information regarding the battery status of the system. The status indicator 2218 can provide information regarding the calibration status of the chemical colorimetric indicator. The status indicator 2218 can provide information regarding whether a cartridge containing a chemical colorimetric indicator is engaged with the system and whether the calibration cartridge is engaged with the system. The status indicator 2218 can provide information regarding the humidification status of the chemical colorimetric indicator. The status indicator 2218 can provide information regarding the need to change the calibration cartridge or the cartridge containing the chemical colorimetric indicator. The status indicator 2218 can also provide notice that the system needs to be re-calibrated. The LED ring 2216 can include one or more different colors. The LED ring 2216 can provide a calming effect on the user of the system. In some embodiments the LED ring 2216 can be used to provide additional feedback to the user as part of the breathing therapy methods described herein. For example, the colors and patterns of light emitted by the LED ring 2216 can provide guidance to the user to help the user vary the breathing rate to match the desired target pattern as part of the breathing therapy. The colors and patterns of light emitted by the LED ring 2216 can also communicate the operational status of the device to the user during startup and calibration. For example, the LED ring 2216 can communicate to the user when the startup and calibration sequences are complete. The LED ring 2216 can also communicate to the user the activity of the calibration process so that the user is aware of the progress of the calibration process. After calibration is complete the LED ring 2216 can provide an indication to the user that the device is ready to provide a breathing therapy. A cannula case 2204 is also illustrated that can be provided for the user. In some embodiments the cannula case 2204 can include a heat sealed pouch.

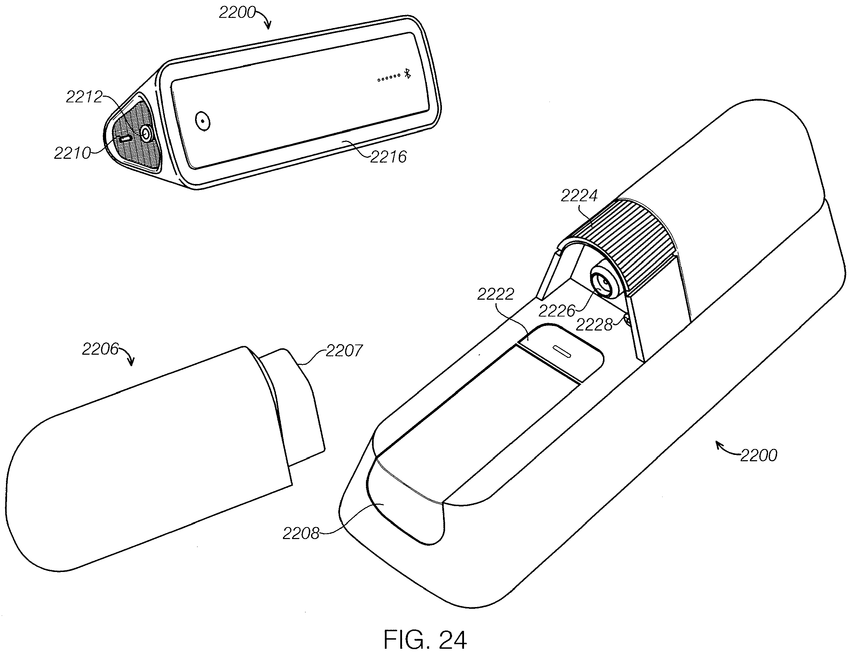

FIG. 24 illustrates a quantitative colorimetric measurement system 2200 including a first removable cartridge 2206 containing carbon dioxide and a second cartridge 2208 containing a chemical colorimetric indicator in accordance with some embodiments. The first removable cartridge 2206 containing the carbon dioxide reference gas is illustrated in a removed position relative to the rest of the system. The second cartridge 2208 containing the chemical colorimetric indicator is shown in a position engaged with the rest of the system. FIG. 24 shows a film release 2222 button that can be depressed to remove the second removable cartridge 2208. A cartridge release button 2224 is also illustrated that can be used to disengage the first removable cartridge 2206. The first removable cartridge 2206 containing the carbon dioxide reference gas includes a complementary engagement surface 2207 that can engage with the system, including the inlets 2226 and 2228. For example a carbon dioxide reference source outlet on the first removable cartridge 2206 can engage with the inlet 2226 to provide the carbon dioxide reference source to the system 2200. A moisture or humidity source outlet on the first removable cartridge 2206 can engage with the inlet 2228 to provide humidification to the system 2200.

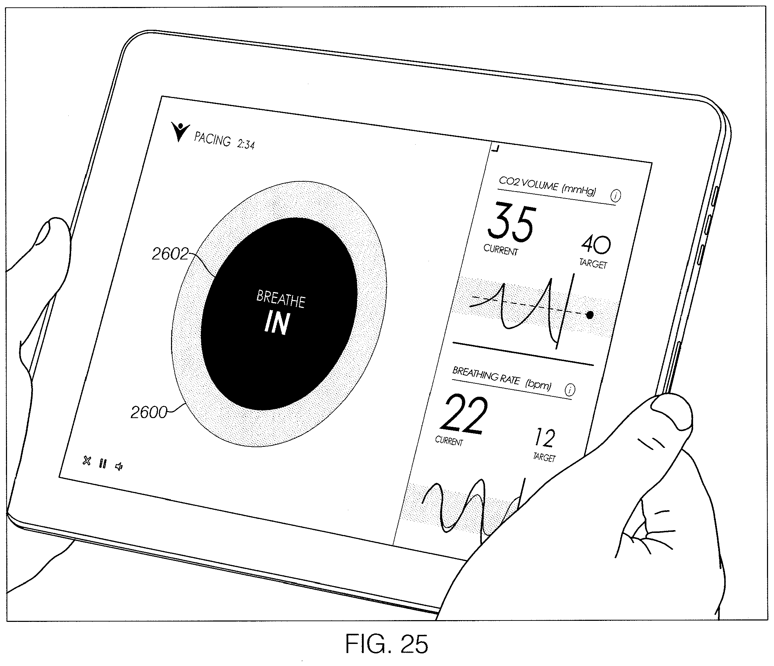

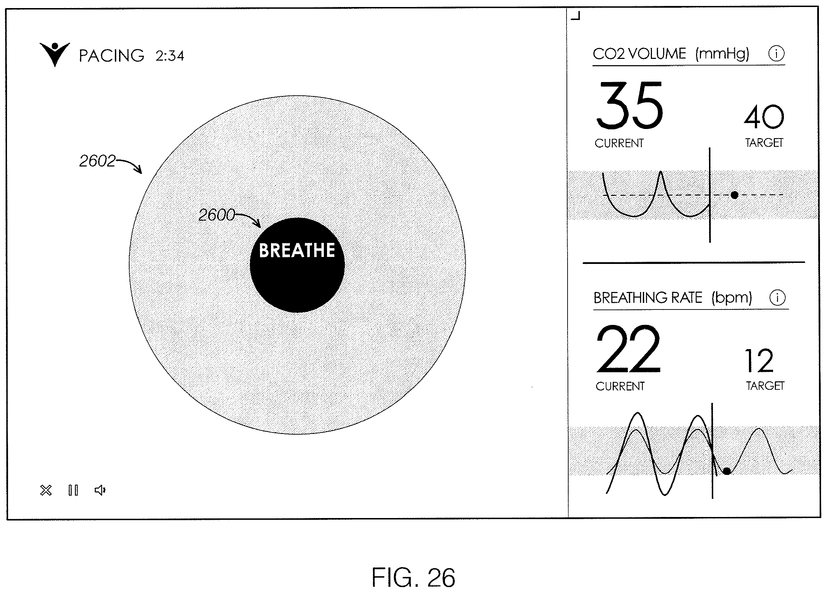

FIG. 25 illustrates an embodiment of a user interface displayed on a handheld computing device and FIG. 26 illustrates an embodiment of a user interface. The user interface shown in FIGS. 25-26 provides a visual pacing indicator on the left side of the user interface. The user interface shown in FIGS. 25-26 provides a visual indicator of the $CO_2$ volume in the exhaled breath along with the breathing rate on the right side of the user interface. The user interface shows the breathing rate and $CO_2$ volume for the user of the system. The user interface can provide the real-time monitoring for each of the breathing rate and $CO_2$ volume along with an indication of the target breathing rate and $CO_2$ volume as well as instructions to help the user reach the target metrics. The visual pacing indicator or visual indication of the target respiration rate on the user interface includes a design with a first circle 2600 having a first diameter and a second circle 2602 having a second diameter that is larger than the first diameter. The second circle provides a pacing guide to the user for the pacing of the inhaling and exhaling during the breathing therapy. The first circle 2600 tracks the inhaling and exhaling of the user during the breathing therapy. The size of the second diameter can change relative to the target pacing, CO2 level, and the user's breathing pattern. For example, to signal to the user to inhale more air the second diameter can be increased. In contrast, to provide a signal to the user to inhale less air, the second diameter can be decreased. During the breathing therapy the second diameter can change relative to the desired signaling for the breathing therapy. Other types of visual cues can be used to represent the difference between the user's exhaled $CO_2$ levels and a target user's exhaled $CO_2$ levels. For example a moving target line or bar graph representation could be used with the location of the target line / bar graph defining a distance between the target line / bar graph and a reference point to represent the difference between the user's exhaled $CO_2$ levels and a target user's exhaled $CO_2$ levels.

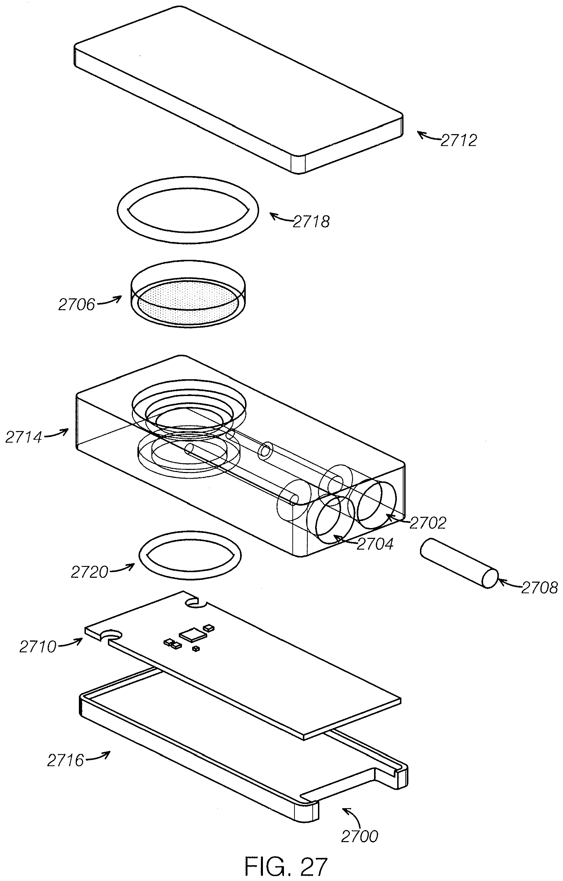

FIG. 27 illustrates an exploded view of a removable cartridge 2700 including a chemical colorimetric indicator 2706 in accordance with some embodiments. The film cartridge 2700 includes an inlet 2702, outlet 2704 and a chemical colorimetric indicator film 2706. A HME filter 2708 is located in the inlet 2702 and can be used to absorb or hold moisture to provide some humidification to the incoming air and the chemical colorimetric indicator film 2706. The film cartridge 2700 includes a printed circuit board (PCB) 2710. The film cartridge 2700 can include a top cover 2712, middle section 2714, and a bottom cover 2716. The film cartridge 2700 includes a first O-ring 2718 and a second O-ring 2720.

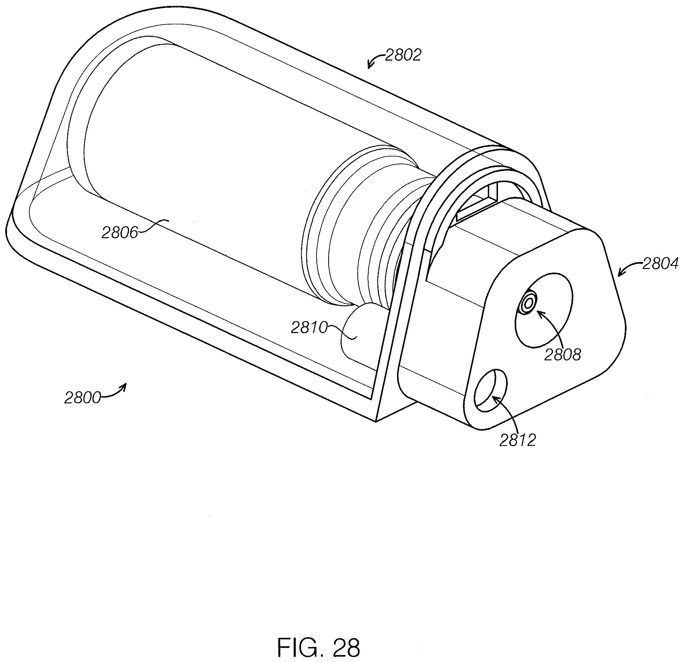

FIG. 28 illustrates a view of a removable cartridge 2800 with a carbon dioxide reference gas in accordance with some embodiments. The removable cartridge 2800 includes an outer housing 2802 and an engagement surface 2804 configured to removably engage with the system. The removable cartridge 2800 includes a $CO_2$ reference source 2806 and a reference gas outlet 2808. The removable cartridge includes a humidity source 2810 and a humidity source outlet 2812.

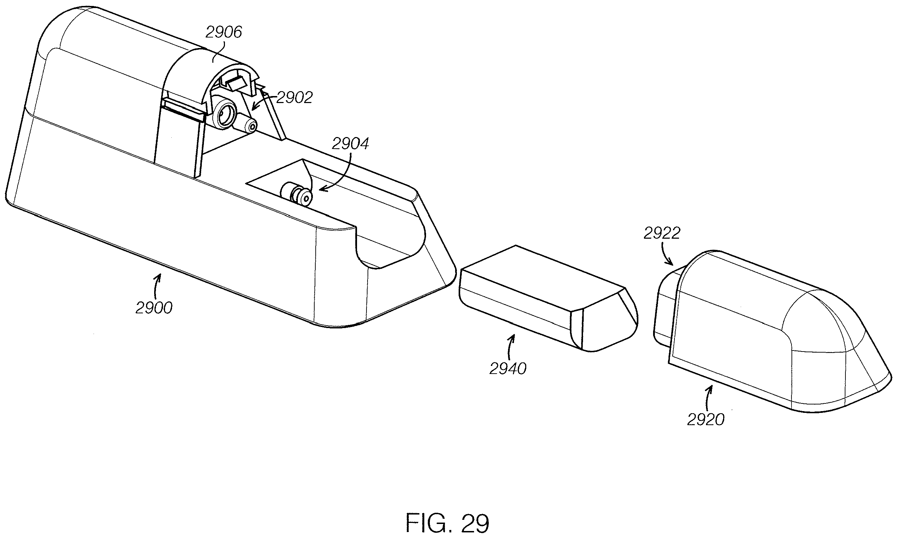

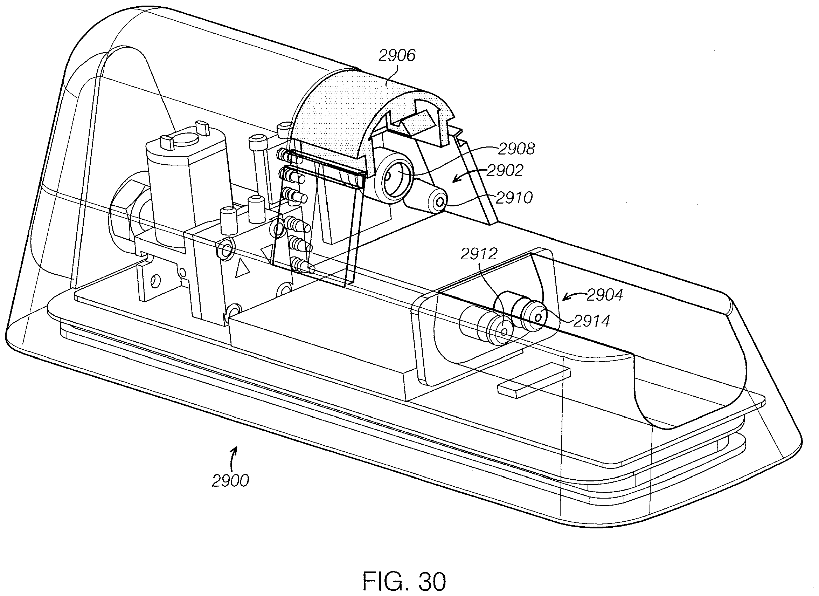

FIG. 29 illustrates a quantitative colorimetric measurement system 2900 including a first removable cartridge 2920 (similar to removable cartridge 2800) containing a carbon dioxide source and a second cartridge 2960 (similar to removable cartridge 2700) containing a chemical colorimetric indicator in accordance with some embodiments. The system 2900 has a complementary engagement surface 2902 configured to engage with an engagement surface 2922 of the first removable cartridge 2920 to form a fluid engagement with the outlets on cartridge 2920. The system 2900 includes a cartridge release 2906 for disengaging the first removable cartridge 2920. The system 2900 has a complementary engagement surface 2904 configured to engage with the second removable cartridge 2940 to form a fluid engagement with the inlet and outlet on cartridge 2940. FIG. 30 illustrates an internal view of the system 2900. The system 2900 includes inlets 2908 and 2910 for engaging with and forming fluid communication with the first removable cartridge 2920. The inlet 2908 can engage with the reference gas outlet 2808 of the removable cartridge 2800 to form a fluid communication with the reference source 2806. The inlet 2910 can engage with the humidity source outlet 2812 of the removable cartridge 2800 to form a fluid communication with the humidity source 2810. The system includes inlets 2912 and 2914. The inlet 2912 can engage with inlet 2702 of film cartridge 2700. The breath sample can pass through inlet 2912 and inlet 2702 to contact the chemical colorimetric indicator 2706. The sample can exit through inlet 2704 and through inlet 2914.

The present application also includes kits including any of the components disclosed herein. In some embodiments kits are provided including a chemical colorimetric indicator configured to be used with a quantitative carbon dioxide measurement system described herein along with any of the characterization and calibration data for the chemical colorimetric indicator described herein. In some embodiments the kits include factory characterization data corresponding to the span calibration performed for the colorimetric indicator. In some embodiments the characterization data includes coefficients for a calibration curve. The calibration curve can be a polynomial equation as described herein. The coefficients can correspond to any of the polynomial equations described herein. In some embodiments the coefficients can include coefficients for a temperature sensitive equation. In some embodiments the calibration curve is a function of a voltage measured by the quantitative carbon dioxide measurement system. In some embodiments the kit can further include a sealed packaging material containing the chemical colorimetric indicator. In some embodiments the packaging material is moisture resistant and light resistant. In some embodiments the characterization data is encoded on the packaging of the chemical colorimetric indicator in a machine readable format.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements, these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1 % of the stated value (or range of values), +/- 2% of the stated value (or range of values), +/- 5% of the stated value (or range of values), +/- 10% of the stated value (or range of values), etc. Any numerical range recited herein is intended to include all subranges subsumed therein.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

**Claims**

1. A quantitative colorimetric measurement system (300) comprising:

   an electro-optical sensor assembly (306) including one or more light sources (410) and a light detection material configured to detect light reflected off of a chemical colorimetric indicator by the light source, the electro-optical sensor assembly configured to generate an electrical signal based on the light detected by the light detection material;
   a humidity sensor (2002) adapted to measure a humidity of the chemical colorimetric indicator;
   a temperature sensor adapted to measure a temperature of the chemical colorimetric indicator;
   a processor (322) configured to determine a quantitative value of $CO_2$ detected by the chemical colorimetric indicator from the humidity of the chemical colorimetric indicator, the temperature of the chemical colorimetric indicator, and a voltage measurement corresponding to the electrical signal based on the light detected by the light detection material; and
   a removable cartridge (1102) comprising the chemical colorimetric indicator and a humidity moisture exchanger (2114), the cartridge configured to removably engage with the quantitative colorimetric measurement system.

2. The system of claim 1, further comprising: a second removable cartridge comprising a humidity source and a reference gas having a known concentration of carbon dioxide, the second cartridge configured to removably engage with the quantitative colorimetric measurement system.

3. The apparatus of claim 1, further comprising a processor in communication with the electro-optical sensor assembly, the processor configured to receive the electrical signals generated by the electro-optical sensor assembly, wherein the processor utilizes the signals to compute the quantity of carbon dioxide exposed to the chemical colorimetric indicator.

4. A method of calibrating a quantitative colorimetric measurement system comprising:

   instructing a user of the quantitative colorimetric measurement system to provide a breath sample to a chemical colorimetric indicator to humidify the chemical colorimetric indicator;
   verifying that the chemical colorimetric indicator has been humidified;
   instructing the user to expose the chemical colorimetric indicator to a first ambient gas;
   measuring a first color of the chemical colorimetric indicator based on the exposure to the first gas;
   instructing the user to expose the chemical colorimetric indicator to a reference gas;
   measuring a second color of the chemical colorimetric indicator based on the exposure to the reference gas; and
   deriving a span calibration based on the difference between the first color of the chemical colorimetric indicator and the second color of the chemical colorimetric indicator.

5. The method of claim 4, wherein the first gas has a concentration of CO2 of about 0% to about 2%.

6. The method of claim 4 or claim 5, wherein the reference gas has a known carbon dioxide concentration of from about 4% to about 7% carbon dioxide.

7. The method of claim 4, further comprising: comparing the span calibration to a factory characterization data for the chemical colorimetric indicator and adjusting a calibration curve for the chemical colorimetric indicator based on the comparison of the factory characterization data and the calibration data.

8. The method of claim 4, wherein the step of measuring the first color of the chemical colorimetric indicator based on the exposure to the first gas comprises reflecting a first light source having a first wavelength off of the chemical colorimetric indicator and detecting a first light reflected from the colorimetric indicator to generate a first measurement signal, the method further comprising: adjusting the first light source properties to align the span calibration with the factory characterization data.

9. A method of determining a quantitative value of $CO_2$ in an exhaled breath sample comprising:

   contacting a chemical colorimetric indicator with the exhaled breath sample;
   measuring a temperature of the chemical colorimetric indicator;
   measuring a humidity of the chemical colorimetric indicator;

measuring a color of the chemical colorimetric indicator based on the exposure to the exhaled breath sample;
obtaining a voltage measurement corresponding to the color of the colorimetric indicator; and
determining the quantitative value of the $CO_2$ in the exhaled breath sample from the humidity of the chemical colorimetric indicator, the temperature of the chemical colorimetric indicator, and the voltage measurement.

10. The method of claim 9, further comprising displaying the user's measured $CO_2$ levels.

11. The method of claim 9 or claim 10, wherein the measured humidity of the chemical colorimetric indicator is applied to the voltage measurement to apply a humidity correction.

**Patentansprüche**

1. Quantitatives kolorimetrisches Messsystem (300), umfassend:

eine elektrooptische Sensoranordnung (306) einschließlich einer oder mehrerer Lichtquellen (410) und eines Lichterfassungsmaterials, das konfiguriert ist, um Licht zu erfassen, das von einem chemischen kolorimetrischen Indikator durch die Lichtquelle reflektiert wird, wobei die elektrooptische Sensoranordnung konfiguriert ist, um ein elektrisches Signal basierend auf dem Licht zu erzeugen, das durch das Lichterfassungsmaterial erfasst wird;
einen Feuchtigkeitssensor (2002), der angepasst ist, um eine Feuchtigkeit des chemischen kolorimetrischen Indikators zu messen;
einen Temperatursensor, der angepasst ist, um eine Temperatur des chemischen kolorimetrischen Indikators zu messen;
einen Prozessor (322), der konfiguriert ist, um einen quantitativen Wert von $CO_2$, erfasst durch den chemischen kolorimetrischen Indikator aus der Feuchtigkeit des chemischen kolorimetrischen Indikators, der Temperatur des chemischen kolorimetrischen Indikators und einer Spannungsmessung, die dem elektrischen Signal entspricht, basierend auf dem Licht, das durch das Lichterfassungsmaterial erfasst wird, zu bestimmen; und
eine entfernbare Patrone (1102), umfassend den chemischen kolorimetrischen Indikator und einen Feuchtigkeitsaustauscher (2114),
wobei die Patrone konfiguriert ist, um mit dem quantitativen kolorimetrischen Messsystem lösbar in Eingriff zu sein.

2. System nach Anspruch 1, ferner umfassend: eine zweite entfernbare Patrone, umfassend eine Feuchtigkeitsquelle und ein Referenzgas mit einer bekannten Konzentration von Kohlendioxid, wobei die zweite Patrone konfiguriert ist, um mit dem quantitativen kolorimetrischen Messsystem lösbar in Eingriff zu sein.

3. Vorrichtung nach Anspruch 1, ferner umfassend einen Prozessor in Verbindung mit der elektrooptischen Sensoranordnung, wobei der Prozessor konfiguriert ist, um die elektrischen Signale zu empfangen, die durch die elektrooptische Sensoranordnung erzeugt werden, wobei der Prozessor die Signale verwendet, um die Menge von Kohlendioxid zu berechnen, die dem chemischen kolorimetrischen Indikator ausgesetzt ist.

4. Verfahren des Kalibrierens eines quantitativen kolorimetrischen Messsystems, umfassend:

Anweisen eines Benutzers des quantitativen kolorimetrischen Messsystems, eine Atemprobe für einen chemischen kolorimetrischen Indikator bereitzustellen, um den chemischen kolorimetrischen Indikator zu befeuchten;
Verifizieren, dass der chemische kolorimetrische Indikator befeuchtet wurde;
Anweisen des Benutzers, den chemischen kolorimetrischen Indikator einem ersten Umgebungsgas auszusetzen;
Messen einer ersten Farbe des chemischen kolorimetrischen Indikators basierend auf dem Aussetzen unter das erste Gas;
Anweisen des Benutzers, den chemischen kolorimetrischen Indikator einem Referenzgas auszusetzen;
Messen einer zweiten Farbe des chemischen kolorimetrischen Indikators basierend auf dem Aussetzen unter das Referenzgas; und
Ableiten einer Spannweitenkalibrierung basierend auf der Differenz zwischen der ersten Farbe des chemischen kolorimetrischen Indikators und der zweiten Farbe des chemischen kolorimetrischen Indikators.

5. Verfahren nach Anspruch 4, wobei das erste Gas eine Konzentration von $CO_2$ von etwa 0 % bis etwa 2 % hat.

6. Verfahren nach Anspruch 4 oder Anspruch 5, wobei das Referenzgas eine bekannte Kohlendioxidkonzentration von etwa 4 % bis etwa 7 % Kohlendioxid hat.

7. Verfahren nach Anspruch 4, ferner umfassend: Vergleichen der Spannweitenkalibrierung mit Fabrikcharakterisierungsdaten für den chemischen kolorimetrischen Indikator und Anpassen einer Kalibrierungskurve für den chemischen kolorimetrischen Indikator basierend auf dem Vergleich der Fabrikcharakterisierungsdaten und der Kalibrierungsdaten.

8. Verfahren nach Anspruch 4, wobei der Schritt des Messens der ersten Farbe des chemischen kolorimetrischen Indikators basierend auf dem Aussetzgen unter das erste Gas das Reflektieren einer ersten Lichtquelle mit einer ersten Wellenlänge von dem chemischen kolorimetrischen Indikator und Erfassen eines ersten Lichts, das von dem kolorimetrischen Indikator reflektiert wird, umfasst, um ein erstes Messsignal zu erzeugen, wobei das Verfahren ferner Folgendes umfasst: Anpassen der ersten Lichtquelleneigenschaften, um die Spannweitenkalibrierung den Fabrikcharakterisierungsdaten anzupassen.

9. Verfahren des Bestimmens eines quantitativen Werts von $CO_2$ in einer Probe aus ausgeatmeter Luft, umfassend:

   Kontaktieren eines chemischen kolorimetrischen Indikators mit der Probe aus ausgeatmeter Luft;
   Messen einer Temperatur des chemischen kolorimetrischen Indikators;
   Messen einer Feuchtigkeit des chemischen kolorimetrischen Indikators;
   Messen einer Farbe des chemischen kolorimetrischen Indikators basierend auf dem Aussetzen unter die Probe aus ausgeatmeter Luft;
   Erhalten einer Spannungsmessung, die der Farbe des kolorimetrischen Indikators entspricht; und
   Bestimmen des quantitativen Werts des $CO_2$ in der Probe aus ausgeatmeter Luft aus der Feuchtigkeit des chemischen kolorimetrischen Indikators, der Temperatur des chemischen kolorimetrischen Indikators und der Spannungsmessung.

10. Verfahren nach Anspruch 9, ferner umfassend das Anzeigen der gemessenen $CO_2$-Werte des Benutzers.

11. Verfahren nach Anspruch 9 oder Anspruch 10, wobei die gemessene Feuchtigkeit des chemischen kolorimetrischen Indikators auf die Spannungsmessung ausgeübt wird, um eine Feuchtigkeitskorrektur auszuüben.

**Revendications**

1. Système de mesure colorimétrique quantitatif (300) comprenant :

   un ensemble capteur électro-optique (306) comprenant une ou plusieurs sources de lumière (410) et un matériau de détection de lumière configuré pour détecter la lumière réfléchie par un indicateur colorimétrique chimique par la source de lumière, l'ensemble capteur électro-optique étant configuré pour générer un signal électrique sur la base de la lumière détectée par le matériau de détection de lumière ;
   un capteur d'humidité (2002) adapté pour mesurer l'humidité de l'indicateur colorimétrique chimique ;
   un capteur de température adapté pour mesurer une température de l'indicateur colorimétrique chimique ;
   un processeur (322) configuré pour déterminer une valeur quantitative de $CO_2$ détectée par l'indicateur colorimétrique chimique à partir de l'humidité de l'indicateur colorimétrique chimique, la température de l'indicateur colorimétrique chimique et une mesure de tension correspondant au signal électrique sur la base de la lumière détectée par le matériau de détection de lumière ; et
   une cartouche amovible (1102), comprenant l'indicateur colorimétrique chimique et un échangeur d'humidité (2114), la cartouche étant configurée pour s'engager de manière amovible avec le système de mesure colorimétrique quantitatif.

2. Système selon la revendication 1, comprenant en outre :
   une deuxième cartouche amovible comprenant une source d'humidité et un gaz de référence ayant une concentration connue de dioxyde de carbone, la deuxième cartouche étant configurée pour s'engager de manière amovible avec le système de mesure colorimétrique quantitatif.

3. Appareil selon la revendication 1, comprenant en outre un processeur en communication avec l'ensemble capteur électro-optique, le processeur étant configuré pour recevoir les signaux électriques générés par l'ensemble capteur électro-optique, dans lequel le processeur utilise les signaux pour calculer la quantité de dioxyde de carbone exposé à l'indicateur colorimétrique chimique.

4. Procédé d'étalonnage d'un système de mesure colorimétrique quantitatif comprenant :

l'instruction d'un utilisateur du système de mesure colorimétrique quantitatif de fournir un échantillon d'haleine à un indicateur colorimétrique chimique pour humidifier l'indicateur colorimétrique chimique ;
la vérification que l'indicateur colorimétrique chimique a été humidifié ;
la demande à l'utilisateur d'exposer l'indicateur colorimétrique chimique à un premier gaz ambiant ;
la mesure d'une première couleur de l'indicateur colorimétrique chimique sur la base de l'exposition au premier gaz ;
la demande à l'utilisateur d'exposer l'indicateur colorimétrique chimique à un gaz de référence ;
la mesure d'une deuxième couleur de l'indicateur colorimétrique chimique sur la base de l'exposition au gaz de référence ; et
la dérivation d'un étalonnage d'étendue basé sur la différence entre la première couleur de l'indicateur colorimétrique chimique et la deuxième couleur de l'indicateur colorimétrique chimique.

5. Procédé selon la revendication 4, dans lequel le premier gaz a une concentration de $CO_2$ d'environ 0% à environ 2%.

6. Procédé selon la revendication 4 ou 5, dans lequel le gaz de référence a une concentration connue en dioxyde de carbone d'environ 4% à environ 7%.

7. Procédé selon la revendication 4, comprenant en outre : la comparaison de l'étalonnage de l'étendue à des données de caractérisation d'usine pour l'indicateur colorimétrique chimique et l'ajustement d'une courbe d'étalonnage pour l'indicateur colorimétrique chimique sur la base de la comparaison des données de caractérisation d'usine et des données d'étalonnage.

8. Procédé selon la revendication 4, dans lequel l'étape de mesure de la première couleur de l'indicateur colorimétrique chimique sur la base de l'exposition au premier gaz comprend la réflexion d'une première source de lumière ayant une première longueur d'onde hors de l'indicateur colorimétrique chimique et la détection d'une première lumière réfléchi par l'indicateur colorimétrique pour générer un premier signal de mesure, le procédé comprenant en outre : l'ajustement des premières propriétés de la source de lumière pour aligner l'étalonnage de l'étendue avec les données de caractérisation d'usine.

9. Procédé de détermination d'une valeur quantitative de $CO_2$ dans un échantillon d'haleine expiré comprenant :

la mise en contact d'un indicateur colorimétrique chimique avec l'échantillon d'haleine expiré ;
la mesure d'une température de l'indicateur colorimétrique chimique ;
la mesure d'une humidité de l'indicateur colorimétrique chimique ;
la mesure d'une couleur de l'indicateur colorimétrique chimique sur la base de l'exposition à l'échantillon d'haleine expiré ;
l'obtention d'une mesure de tension correspondant à la couleur de l'indicateur colorimétrique ; et
la détermination de la valeur quantitative de $CO_2$ dans l'échantillon d'haleine expirée à partir de l'humidité de l'indicateur colorimétrique chimique, de la température de l'indicateur colorimétrique chimique et de la mesure de tension.

10. Procédé selon la revendication 9, comprenant en outre l'affichage des niveaux de $CO_2$ de l'utilisateur mesurés.

11. Procédé selon la revendication 9 ou 10, dans lequel l'humidité mesurée de l'indicateur colorimétrique chimique est appliquée à la mesure de tension pour appliquer une correction d'humidité.

FIG. 1

FIG. 2

_200_

_201_ CO2 → _202_ CONDUIT → _204_ COLORIMETRIC INDICATOR → _206_ PUMP → _208_ EXIT

COLORIMETRIC INDICATOR → _210_ ELECTRO-OPTICAL SENSOR ASSEMBLY → _212_ MEASUREMENT SIGNAL

MEASUREMENT SIGNAL → _214_ ON BOARD PROCESSOR → _216_ DISPLAY

MEASUREMENT SIGNAL → _218_ TRANSMIT MEASUREMENT SIGNAL → _220_ EXTERNAL PROCESSOR → _222_ DISPLAY

FIG. 3A

250

251

C02

252

CONDUIT

254

PUMP

256

COLORIMETRIC
INDICATOR

258

EXIT

260

ELECTRO-OPTICAL
SENSOR ASSEMBLY

266

DISPLAY

264

ON BOARD
PROCESSOR

262

MEASUREMENT
SIGNAL

272

DISPLAY

270

EXTERNAL
PROCESSOR

268

TRANSMIT MEASUREMENT
SIGNAL

FIG. 3B

FIG. 4

EP 3 304 494 B1

500

502

HUMIDIFYING A CHEMICAL COLORIMETRIC INDICATOR

504

EXPOSING THE CHEMICAL COLORIMETRIC INDICATOR TO A FIRST GAS

506

MEASURING A FIRST COLOR OF THE CHEMICAL COLORIMETRIC INDICATOR BASED ON THE EXPOSURE TO THE FIRST GAS

508

EXPOSING THE CHEMICAL COLORIMETRIC INDICATOR TO A SECOND GAS HAVING A DIFFERENT $CO_2$ CONCENTRATION THAN THE FIRST GAS

510

MEASURING A SECOND COLOR OF THE CHEMICAL COLORIMETRIC INDICATOR BASED ON THE EXPOSURE TO THE SECOND GAS

512

DERIVING A SPAN $CO_2$ CALIBRATION BASED ON THE DIFFERENCE BETWEEN THE FIRST COLOR OF THE CHEMICAL COLORIMETRIC INDICATOR AND THE SECOND COLOR OF THE CHEMICAL COLORIMETRIC INDICATOR

FIG. 5

600

602
INSTRUCTING A USER OF THE QUANTITATIVE COLORIMETRIC DETECTION SYSTEM TO PROVIDE A BREATH SAMPLE TO A CHEMICAL COLORIMETRIC INDICATOR TO HUMIDIFY THE CHEMICAL COLORIMETRIC INDICATOR

604
VERIFYING THAT THE CHEMICAL COLORIMETRIC INDICATOR HAS BEEN HUMIDIFIED

606
INSTRUCTING THE USER TO EXPOSE THE CHEMICAL COLORIMETRIC INDICATOR TO A FIRST AMBIENT GAS

608
MEASURING A FIRST COLOR OF THE CHEMICAL COLORIMETRIC INDICATOR BASED ON THE EXPOSURE TO THE FIRST GAS

610
INSTRUCTING THE USER TO EXPOSE THE CHEMICAL COLORIMETRIC INDICATOR TO A REFERENCE GAS

612
MEASURING A SECOND COLOR OF THE CHEMICAL COLORIMETRIC INDICATOR BASED ON THE EXPOSURE TO THE REFERENCE GAS

614
DERIVING A SPAN CALIBRATION BASED ON THE DIFFERENCE BETWEEN THE FIRST COLOR OF THE CHEMICAL COLORIMETRIC INDICATOR AND THE SECOND COLOR OF THE CHEMICAL COLORIMETRIC INDICATOR

FIG. 6

700

702

CONTACTING THE CHEMICAL COLORIMETRIC INDICATOR SEQUENTIALLY WITH A PLURALITY OF GASES HAVING A PLURALITY OF DIFFERENT CONCENTRATIONS OF CARBON DIOXIDE

704

MEASURING A COLOR OF THE CHEMICAL COLORIMETRIC INDICATOR BASED ON AN EXPOSURE TO EACH OF THE PLURALITY OF GASES BY REFLECTING A FIRST LIGHT SOURCE HAVING A FIRST WAVELENGTH OFF OF THE CHEMICAL COLORIMETRIC INDICATOR AND DETECTING A FIRST LIGHT REFLECTED FROM THE COLORIMETRIC INDICATOR TO GENERATE A PLURALITY OF MEASUREMENT SIGNALS

706

DETERMINING A CHARACTERIZATION FOR THE CHEMICAL COLORIMETRIC INDICATOR BY ANALYZING THE PLURALITY OF MEASUREMENT SIGNALS CORRESPONDING TO THE PLURALITY OF DIFFERENT CONCENTRATIONS OF CARBON DIOXIDE

708

CALCULATING A CALIBRATION CURVE FOR THE CHEMICAL COLORIMETRIC INDICATOR BASED ON THE CHARACTERIZATION FOR THE CHEMICAL COLORIMETRIC INDICATOR

FIG. 7A

800

┌─────────────────────────────────────────────────────────────────┐ 802
│ EXPOSING THE CHEMICAL COLORIMETRIC INDICATOR TO A FIRST          │
│ GAS HAVING A FIRST CONCENTRATION OF CARBON DIOXIDE               │
└─────────────────────────────────────────────────────────────────┘

┌─────────────────────────────────────────────────────────────────┐ 804
│ MEASURING A COLOR OF THE CHEMICAL COLORIMETRIC INDICATOR BASED ON THE EXPOSURE TO THE │
│ FIRST GAS BY REFLECTING A FIRST LIGHT SOURCE HAVING A FIRST WAVELENGTH OFF OF THE CHEMICAL │
│ COLORIMETRIC INDICATOR AND DETECTING A FIRST LIGHT REFLECTED FROM THE COLORIMETRIC │
│ INDICATOR TO GENERATE A FIRST MEASUREMENT SIGNAL │
└─────────────────────────────────────────────────────────────────┘

┌─────────────────────────────────────────────────────────────────┐ 806
│ EXPOSING THE CHEMICAL COLORIMETRIC INDICATOR TO A SECOND GAS HAVING A SECOND │
│ CONCENTRATION OF CARBON DIOXIDE │
└─────────────────────────────────────────────────────────────────┘

┌─────────────────────────────────────────────────────────────────┐ 808
│ MEASURING THE COLOR OF THE CHEMICAL COLORIMETRIC INDICATOR BASED ON THE EXPOSURE TO THE │
│ SECOND GAS BY REFLECTING THE FIRST LIGHT SOURCE HAVING THE FIRST WAVELENGTH OFF OF THE │
│ CHEMICAL COLORIMETRIC INDICATOR AND DETECTING THE SECOND LIGHT REFLECTED FROM THE │
│ COLORIMETRIC INDICATOR TO GENERATE A SECOND MEASUREMENT SIGNAL │
└─────────────────────────────────────────────────────────────────┘

┌─────────────────────────────────────────────────────────────────┐ 810
│ DETERMINING A SPAN CO2 CALIBRATION BASED ON THE DIFFERENCE BETWEEN THE FIRST MEASUREMENT │
│ SIGNAL AND THE SECOND MEASUREMENT SIGNALS │
└─────────────────────────────────────────────────────────────────┘

┌─────────────────────────────────────────────────────────────────┐ 812
│ COMPARING THE SPAN CALIBRATION TO A FACTORY CHARACTERIZATION DATA │
│ FOR THE CHEMICAL COLORIMETRIC INDICATOR │
└─────────────────────────────────────────────────────────────────┘

FIG. 7B

FIG. 8

EP 3 304 494 B1

CO2 TO VOLTAGE

FIG. 9A

VOLTAGE TO CO2

FIG. 9B

C02 TO VOLTAGE

FIG. 9C

FIG. 9D

| a | b | c |
|---|---|---|
| 0.0000 | -0.0022 | 0.0197 |
| -0.0000 | 0.0111 | -0.3017 |
| 0.0022 | -0.0813 | 1.7052 |
| -0.0027 | 0.1142 | -2.1403 |

FIG. 9E

900

902

RECEIVING AT LEAST A PORTION OF A USER'S EXHALED AIR IN A GAS INLET OF A QUANTITATIVE COLORIMETRIC DETECTION SYSTEM

904

MEASURING THE HUMIDITY OF A HUMIDITY SENSOR WITHIN THE QUANTITATIVE COLORIMETRIC DETECTION SYSTEM THAT IS EXPOSED TO THE USER'S EXHALED AIR

906

CONFIRMING THAT THE HUMIDITY OF THE HUMIDITY SENSOR IS ABOVE A THRESHOLD HUMIDITY

908

MEASURING A USER'S END-TIDAL CO2 LEVELS WITH THE QUANTITATIVE COLORIMETRIC DETECTION SYSTEM BASED ON A COLOR CHANGE RESULTING FROM EXPOSURE OF THE SYSTEM TO THE USER'S EXHALED AIR

FIG. 10A

950 ⟍

952

HUMIDIFYING A CHEMICAL COLORIMETRIC INDICATOR IN A QUANTITATIVE COLORIMETRIC DETECTION SYSTEM BY PASSING A GAS THROUGH A HUMIDITY SOURCE AND INTO CONTACT WITH THE CHEMICAL COLORIMETRIC INDICATOR

954

MEASURING THE HUMIDITY OF A HUMIDITY SENSOR WITHIN THE QUANTITATIVE COLORIMETRIC DETECTION SYSTEM THAT IS EXPOSED TO THE USER'S EXHALED AIR

956

RECEIVING AT LEAST A PORTION OF A USER'S EXHALED AIR IN A GAS INLET OF A QUANTITATIVE COLORIMETRIC DETECTION SYSTEM AND CONTACTING THE CHEMICAL COLORIMETRIC INDICATOR WITH THE PORTION OF THE USER'S EXHALED AIR

958

MEASURING A USER'S END-TIDAL CO2 LEVELS WITH THE QUANTITATIVE COLORIMETRIC DETECTION SYSTEM BASED ON A COLOR CHANGE RESULTING FROM EXPOSURE OF THE SYSTEM TO THE USER'S EXHALED AIR

FIG. 10B

FIG. 11

EP 3 304 494 B1

FIG. 12A

EP 3 304 494 B1

FIG. 12B

FIG. 13

FIG. 14

FIG. 15A

EP 3 304 494 B1

FIG. 16

FIG. 17A

FIG. 17B

FIG. 17C

FIG. 18

FIG. 19

FIG. 20

FIG. 21

EP 3 304 494 B1

FIG. 22

2200

2218

2220

2212

2214

2210

2216

2204

FIG. 23

EP 3 304 494 B1

EP 3 304 494 B1

FIG. 24

FIG. 25

EP 3 304 494 B1

FIG. 26

EP 3 304 494 B1

FIG. 27

FIG. 28

FIG. 29

EP 3 304 494 B1

FIG. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015009792 A

**Non-patent literature cited in the description**

- A New Colorimetric Breath Indicator (Colibri). *Anesthesia,* 1994, vol. 49, 798-803